# FASCICULE DE BREVET EUROPEEN

(11) **EP 1 355 613 B1**
(45) Date de publication et mention de la délivrance du brevet: **06.06.2007**
(21) Numéro de dépôt: 01270302.1
(22) Date de dépôt: 22.11.2001
(51) Int. Cl.: A61Q 1/00, A61Q 1/02, A61Q 1/12, A61K 8/19, A61K 8/25, A61K 8/29, A61K 8/72, A61K 8/89, A61K 8/92, B05B 7/24, B65D 83/14

(54) **DISPOSITIF POUR LA PULVERISATION D'UN PRODUIT COSMETIQUE**
VORRICHTUNG ZUM ZERSTÄUBEN EINES KOSMETISCHEN PRODUKTES
DEVICE FOR SPRAYING A COSMETIC PRODUCT

(30) Priorité: 15.12.2000 FR 0016397
(43) Date de publication de la demande: 29.10.2003
(73) Titulaire: L'ORÉAL, 75008 Paris (FR)
(72) Inventeur: BARA, Isabelle, F-94210 La Varenne St. Hilaire (FR)
(74) Mandataire: Tanty, François
(86) Numéro de dépôt international: PCT/FR2001/003689
(87) Numéro de publication internationale: WO 2002/047618

(56) Documents cités:
- EP-A- 1 004 288
- FR-A- 2 781 208
- FR-A- 2 785 594
- GB-A- 780 885
- US-A- 5 082 652
- US-A- 5 810 252
- US-A- 5 945 085

## Description

La présente invention concerne un dispositif portatif autonome pour la pulvérisation d'un produit cosmétique sur un support kératinique, notamment la peau, les lèvres ou les ongles.

L'utilisation d'un jet de gaz vecteur pour pulvériser une peinture est connue depuis longtemps, notamment par le brevet US 2,635,921. Ce principe a été largement développé et a fait l'objet de nombreux dépôts de brevets. A titre indicatif, on peut citer la demande de brevet européen EP-Al-0 208 247 ou les brevets US 5,255,852 et 5,713,519.

On connaît également par le brevet US 1,430,506 un dispositif comportant un réservoir sur lequel sont fixées deux buses reliées à une arrivée d'air comprimé, ces deux buses pouvant émettre des jets d'air se rencontrant dans une région située au-dessus d'un orifice d'arrivée du produit. Un tel dispositif requiert une source extérieure d'air comprimé avec un flexible d'alimentation.

Il est connu d'appliquer un produit de maquillage au moyen d'un aérographe encore appelé air-brush comportant un stylo pulvérisateur relié à une bouteille d'un gaz vecteur comprimé, ce stylo pulvérisateur comprenant d'une part une tuyère d'éjection du gaz vecteur et d'autre part une coupelle dans laquelle le produit à pulvériser est contenu, ce produit étant progressivement aspiré par un conduit dans la coupelle grâce à la dépression créée par effet Venturi par la vitesse du gaz vecteur à sa sortie de la tuyère.

La pulvérisation de type air-brush permet notamment de rendre moins visible la frontière entre les zones traitées et non traitées, de réaliser des fondus, des dégradés ou des impressions de volume.

La gestuelle est également différente, puisque le produit qui est pulvérisé n'a pas à être étalé une fois déposé sur le support kératinique.

L'application du produit est ainsi très hygiénique, puisqu'elle peut se faire sans les doigts et sans applicateur.

Un dispositif tel que décrit ci-dessus fait l'objet par exemple de la demande de brevet canadien CA-A-2 152 406.

Des dispositifs similaires sont commercialisés par la société DINAIR sous les marques déposées BEAUTY ART ou BODY ART, la bouteille de gaz vecteur comprimé étant remplacée par un compresseur à air.

Ces dispositifs connus, dans lesquels le stylo pulvérisateur et la source de gaz vecteur ne constituent pas une construction unitaire (self-contained), étant reliés par un flexible, sont relativement encombrants et sont destinés principalement à un usage professionnel. Ils ne conviennent pas pour être transportés aisément par une femme dans son sac à main, à l'instar des accessoires de maquillage conventionnels.

Par ailleurs, on connaît par la demande de brevet français FR-A-2 781 208 un dispositif portatif autonome de pulvérisation d'un produit de maquillage dans lequel le gaz vecteur est présent sous une forme liquéfiée dans un récipient, le produit à pulvériser et le gaz vecteur étant stockés séparément.

Lorsque l'on utilise un gaz vecteur stocké à l'état liquéfié, l'autonomie est accrue et le refroidissement qui accompagne la détente du gaz vecteur contribue à procurer une sensation de fraîcheur au moment de l'application.

Le produit peut être amené au contact du gaz vecteur au moyen d'un conduit ou par capillarité au moyen d'un feutre.

Dans un tel dispositif portatif autonome, la pulvérisation du produit soulève de nombreuses difficultés.

Tout d'abord, le débit avec lequel le produit est amené au contact du gaz vecteur pour être entraîné par celui-ci ne doit pas être trop important car sinon le spray risque de présenter des gouttelettes ayant une taille trop grande ou irrégulières, ni trop faible car la consommation de gaz vecteur augmente alors rapidement et l'autonomie du dispositif devient insuffisante.

Un feutre est souvent utilisé pour amener le produit au contact du gaz vecteur, car il permet un débit de produit répondant assez bien à ces exigences contradictoires.

Toutefois, l'utilisation d'un feutre n'est pas toujours souhaitable lorsque le produit contient une proportion importante de particules solides car le feutre est susceptible de se comporter comme un filtre retenant les particules solides.

Lorsque le feutre est remplacé par un conduit, soit parce que le produit est trop visqueux ou contient une proportion trop importante de particules solides, un bouchage de ce conduit risque de se produire si sa section est trop faible. La section du conduit utilisé ne doit cependant pas être trop grande au risque de conduire à un spray ayant des gouttelettes trop grosses ou de taille irrégulière, ce qui nuirait à la qualité du maquillage.

Le brevet US 4 742 963 décrit un autre dispositif portatif autonome dans lequel le gaz vecteur et le produit sont stockés dans le même récipient au contact l'un de l'autre. Il est prévu de secouer si nécessaire le récipient afin d'homogénéiser le produit avant l'utilisation du dispositif. Dans ce dispositif connu, le produit à pulvériser n'est pas aspiré par une dépression créée par effet Venturi mais envoyé sous pression dans une tuyère d'éjection. Les problèmes précités ne se posent pas mais du produit est susceptible d'encrasser la tuyère d'éjection, du fait de l'absence de gaz vecteur.

La demande de brevet japonais JP-A-63287711 décrit une émulsion cosmétique destinée à être pulvérisée, contenant de la lécithine destinée à rendre l'application plus uniforme. Cette demande ne traite pas des difficultés mentionnées plus haut rencontrées avec les dispositifs portatifs autonomes tels que celui décrit dans la demande FR-A-2 781 208.

Il existe par conséquent un besoin pour bénéficier d'un dispositif portatif autonome capable de pulvériser un produit comportant au moins 0,3 % en poids de particules solides, avec de bonnes propriétés de spray et capable de procurer un bon résultat de maquillage, dispositif pour lequel, notamment, le risque de dysfonctionnement est réduit.

En particulier, il existe notamment un besoin pour bénéficier d'un dispositif portatif autonome dans lequel le produit est pulvérisé grâce à l'aspiration provoquée par effet Venturi par un gaz vecteur, qui puisse fonctionner avec une autonomie compatible avec les attentes des utilisatrices et avec un produit comportant une proportion relativement élevée de particules solides, tel qu'un fond de teint, sans risque de bouchage du conduit d'amenée du produit même en cas de stockage du dispositif pendant une période prolongée et d'absence d'agitation ou d'agitation modérée du produit au moment de l'utilisation.

Le nouveau dispositif autonome de construction unitaire selon l'invention permet notamment de répondre à ce besoin et se caractérise par le fait qu'il comprend une réserve de produit à pulvériser et un récipient contenant un gaz vecteur, ainsi qu'une valve permettant, lorsqu'elle est actionnée, la pulvérisation du produit, ce dernier contenant des particules solides et au moins un gélifiant, dispositif dans lequel la réserve de produit est contenue dans un récipient différent de celui contenant le gaz ou le produit est contenu dans une poche souple placée à l'intérieur du récipient contenant le gaz.

Grâce à la présence au sein du produit contenu dans le dispositif portatif autonome, en plus des particules solides, d'un gélifiant, il est possible de faire en sorte que le produit soit suffisamment fluide pour pouvoir être prélevé, notamment par aspiration par effet Venturi au moyen d'un gaz vecteur, tout en empêchant les particules solides de s'agglomérer d'une manière telle que cela pourrait provoquer le bouchage du conduit par lequel le produit à pulvériser est amené dans la zone en dépression.

La présence d'un gélifiant permet notamment d'éviter ou de ralentir la sédimentation ou l'apparition d'une nouvelle phase ou de crème au sein du produit lors de son stockage, et favorise donc un prélèvement homogène du produit tout au long du fonctionnement du dispositif.

Dans une mise en oeuvre particulière de l'invention, le produit comporte et au moins un gélifiant et au moins un dispersant.

De préférence, le dispersant ou gélifiant est choisi de manière à ne pas cristalliser dans le milieu qui le contient à température ambiante et dans les conditions d'utilisation.

La température ambiante s'entend de la plage de températures correspondant aux conditions extrêmes d'utilisation, typiquement entre 10 et 45 °C.

Parmi les gélifiants ou dispersants aptes à cristalliser dans le milieu à température ambiante et que de préférence, on n'utilisera pas pour mettre en oeuvre l'invention, on peut citer :
- les cires, notamment les cires hydrocarbonées ou les cires de silicone (alkyle ou alcoxydiméticone),
- les esters d'alcool gras et d'acide gras, dont les cérides et les stérides ou les esters d'acides gras et de glycérophoscholine, tels que les phospholipides,
- les acides gras et aminés notamment les céramides.

Le produit peut comporter plus de 0,3 % de particules solides, par exemple entre 5 et 70 % en poids de particules solides, de préférence entre 20 et 70 %, de préférence encore entre 30 et 60 % lorsqu'il s'agit d'un fond de teint, notamment, le dispersant ou le gélifiant étant de préférence dans une proportion comprise entre 0,01 et 10 % en poids, de préférence supérieure ou égale à 0,1 %.

Les particules contenues dans le produit peuvent être colorées ou non.

Dans une mise en oeuvre particulière de l'invention, le produit comporte des pigments, éventuellement plusieurs pigments de couleurs différentes.

Lorsque le produit comporte des pigments de couleurs différentes, la présence d'au moins un gélifiant permet de réduire la séparation des pigments d'une couleur donnée par rapport aux pigments d'une autre couleur, ce qui permet d'obtenir un maquillage ayant une tonalité constante dans le temps.

Les particules peuvent être des pigments tels que par exemple l'oxyde de fer, de titane ou de zinc, des laques organiques ou des nacres minérales, de mica, de titane ou de micatitane, par exemple.

Les particules peuvent être encore des poudres organiques, notamment des particules de polyamide, de polyéthylène, de polyacrylate, de méthacrylate de méthyle, de polyuréthane, de polystyrène réticulé, ou de leurs mélanges, par exemple.

Les particules peuvent encore être des poudres d'origine minérale telles que le talc, l'argile, la silice, le nitrure de bore ou leurs mélanges, ou des poudres d'origine végétale telles que l'amidon, ou des poudres d'origine animale telles que la poudre de coquillages.

Les gélifiants peuvent être modifiés le cas échéant par des groupements organiques afin de gélifier des huiles.

Les particules peuvent encore être des silicones sous forme de billes de résine methylsesquioxane telles que celles commercialisées sous la dénomination commerciale "TOSPEARL" par la société TOSHIBA, ou des silicones réticulés, par exemple sous forme de dispersion aqueuse comme décrit dans le brevet US 5 928 660 notamment.

Les particules peuvent encore provenir de poudres fluorées telles que des poudres de PTFE (polytétrafluoroéthylène).

Les particules peuvent être de taille nanométrique, et comporter par exemple des nanotitanes ou nanooxydes de fer, ou de taille micrométrique. Le produit peut comporter à la fois des particules de tailles micrométrique et nanométrique.

Les particules peuvent être enrobées ou non.

Le produit comporte de préférence, comme mentionné plus haut, entre 0,01 et 10 % en poids de gélifiant(s), en fonction de la nature des autres composés du produit, de telle sorte que la viscosité du produit soit compatible avec le mode de prélèvement du produit dans la réserve de produit, notamment un prélèvement par aspiration grâce à l'effet Venturi.

Le ou les dispersant(s) et gélifiant(s) utilisé(s) peuvent être hydrophiles ou lipophiles.

On peut utiliser notamment, comme gélifiant, des gélifiants hydrophiles de nature polymérique tels que par exemple des gélifiants de type polysaccharide, par exemple la gomme de xanthane, de gellane, de guar ou ses dérivés, la cellulose et ses dérivés, des gélifiants de type vinylique, carboxyvinylique, acrylique, le polyuréthane, des gélifiants de nature non polymérique de type minéral comme les argiles, par exemple le Veegum® commercialisé par la société VANDERBILT, la Bentone® commercialisée par la société RHEOX modifiée ou non ou des silices hydrophiles.

On peut utiliser notamment comme gélifiants lipophiles des gélifiants type polymère ou de type "organo gélateur", c'est-à-dire sous forme de molécules de petite taille non polymérique établissant des interactions de manière à constituer des réseaux, tels que ceux cités dans le livre "Specialist Surfactants" de Blackie Academic et Professional, chapitre 8.

Comme gélifiant de type organogélateur on peut citer l'acide 12 hydroxystéarique, ses sels et dérivés (esters ou amides), les amides amino acides ou de N-acidaminoacides, les amides d'acides tricarboxyliques, le dibenzylidène sorbitol ou alditol et leurs dérivés.

Comme exemple de polymères gélifiants lipophiles, on peut citer les polymères siliconés et leurs dérivés (PDMS de haut poids moléculaire supérieur à 10 000), ces polymères siliconés pouvant être linéaires ("gomme" telle que contenue dans le produit de référence Q2 1401 de la société DOW CORNING ou réticulés de type "résine" tels que ceux contenus dans le produit de référence KSG 6 ou 16 de la société SHIN ETSU ou ceux connus sous la référence TREFIL 505 C ou 506 C de la société DOW CORNING ou encore ceux connus sous la référence SR 5 CYC de la société GRANSIL.

On peut également utiliser comme gélifiants lipophiles de type polymère des polymères dérivés des silicones ou des silicones acryliques.

On peut encore utiliser comme polymères gélifiants lipophiles des polycondensats de type polyamides ou des polysaccharides à chaînes hydrophobes ou des polyuréthanes.

Lorsqu'un ou plusieurs dispersants sont utilisés, ceux-ci seront de type hydrophile ou lipophile et agiront par répulsion électrostatique ou stabilisation stérique.

Comme dispersants hydrophiles, on peut citer notamment ceux ayant au moins une charge anionique, par exemple les dispersants carboxyliques, les sulfonates, les terpolymères de l'acide acrylique, les polyaspartates, les dérivés de l'acide maléique, ces dispersants agissant plutôt par répulsion électrostatique.

Parmi les dispersants qui agissent plutôt par stabilisation stérique, on peut citer les dispersants de type PVP (polyvinyle pyrrolidone), le polyoxyéthylène ou le polycaprolactone.

Les dispersants utilisés en milieu huileux pourront être des carboxylates, des acryliques, des hydrocarbonés, avec cycle, par exemple styrène, ou de type succinimide.

Les gélifiants ou dispersants listés ci-avant peuvent être utilisés en mélange.

Le produit peut comporter au moins un dispersant et au moins un gélifiant.

D'une manière générale, la réserve de produit est avantageusement contenue dans un récipient suffisamment clos pour permettre une conservation du produit dans le dispositif, en l'absence d'utilisation, supérieure à au moins un mois à température ambiante.

La réserve de produit est avantageusement contenue dans une cartouche amovible.

En variante, la réserve de produit peut être contenue dans un récipient non amovible.

De préférence, la viscosité du produit est comprise entre 20 centipoises et 500 centipoises, c'est-à-dire entre 20 millipascals/s et 500 millipascals/s, la viscosité étant mesurée à l'aide d'un rhéomate RM 180 de la société RHEOMETRIC SCIENTIFIC, de type rotatif, utilisant un mobile adapté dit de type "1", "2" ou "3" selon la fluidité de la formule, après dix minutes d'un taux de cisaillement de 200/s, la température des mesures étant de 25° C.

Le produit peut comporter par ailleurs tous les composés usuels utilisés habituellement en cosmétique, notamment des actifs cosmétiques hydrophiles ou lipophiles tels que par exemple des agents bloqueurs d'UV (des filtres) ou des hydratants (la glycérines par exemple), des huiles de soin, des anti-oxydants, des conservateurs, des parfums ou des agents anti-mousse, cette liste n'étant pas exhaustive.

Le dispositif peut comporter un tube plongeant dans la réserve de produit.

Le produit peut être contenu dans une poche souple placée à l'intérieur d'un récipient contenant le gaz.

En variante, la réserve de produit peut être contenue dans un récipient différent de celui contenant le gaz.

Le récipient peut être équipé d'une valve à au moins trois positions, à savoir une position de repos, une position permettant la distribution du produit et du gaz propulseur ou vecteur, et une position permettant la distribution de gaz vecteur uniquement.

Le dispositif peut comporter au moins un conduit d'amenée du produit et au moins un conduit d'amenée du gaz vecteur, de préférence deux, les conduits étant agencés de telle sorte que la sortie de gaz vecteur par le conduit d'amenée du gaz vecteur provoque une dépression apte à entraîner par aspiration du produit provenant du conduit d'amenée de produit.

Le dispositif peut comporter une réserve de gaz vecteur, au moins une arrivée de produit apte à être mise en communication fluidique avec une réserve du produit, ce dernier étant aspiré dans la réserve grâce à une dépression créée dans le voisinage de ladite au moins une arrivée de produit au moyen d'une émission de gaz vecteur.

Le dispositif peut comporter au moins un obturateur apte à interrompre la communication fluidique entre ladite au moins une arrivée de produit et la réserve de produit.

On peut ainsi améliorer les conditions de conservation du produit dans la réserve de produit et notamment éviter l'échappement de composants volatils.

On peut également d'éviter les risques de fuite de produit lorsque le dispositif est transporté en étant allongé ou à l'envers, ce qui est le cas par exemple dans un sac à main.

L'arrivée de produit peut comporter au moins un orifice.

Le dispositif peut être agencé de manière à rétablir automatiquement une communication fluidique entre ladite au moins une arrivée de produit et la réserve de produit, lors d'une émission de gaz vecteur. Le dispositif peut ainsi être agencé de manière à ce que la communication fluidique soit automatiquement interrompue lorsque cesse l'émission de gaz vecteur.

Le dispositif peut comporter un bouton-poussoir apte à agir simultanément, directement ou indirectement, sur une valve de distribution de gaz vecteur et sur l'obturateur, de manière à ce qu'une communication fluidique entre l'arrivée de produit et la réserve de produit soit établie quand l'utilisateur appuie sur le bouton-poussoir pour délivrer du gaz vecteur.

La réserve de produit peut être contenue par exemple dans un premier récipient fixé sur un deuxième récipient contenant le gaz vecteur. Les deux récipients peuvent également avoir au moins une partie commune, par exemple une cloison définissant au moins deux compartiments contenant respectivement le gaz vecteur et le produit à pulvériser.

Le récipient contenant le produit et le récipient contenant le gaz vecteur peuvent être liés fixement, sans déplacement de l'un par rapport à l'autre au cours de l'utilisation. On peut obtenir par exemple de cette façon un ensemble relativement compact, aisé à transporter, dans un sac à main notamment.

Le récipient contenant le produit peut présenter par exemple une forme annulaire, afin de ménager un passage, par exemple central, dans lequel peut s'étendre un organe de commande d'une valve équipant le récipient contenant le gaz vecteur.

L'obturateur peut être relié opérationnellement à au moins un élément actionné par le déplacement d'un bouton-poussoir du dispositif. Un tel élément peut comporter par exemple une tige creuse, ayant au moins un canal intérieur permettant au produit contenu dans la réserve de produit de gagner l'arrivée de produit.

L'obturateur peut comporter par exemple au moins un joint, apte à obturer au moins un orifice par lequel le produit peut s'écouler pour gagner ladite au moins une arrivée de produit et apte à libérer cet orifice au moins partiellement lors de la distribution du produit.

Dans une réalisation particulière, l'obturateur est constitué par un joint monté sur une tige creuse, cette dernière étant fermée à son extrémité inférieure, le joint pouvant venir en appui par sa face supérieure contre un épaulement de la tige. Cette dernière est traversée par exemple par au moins un orifice d'admission de produit, dont le diamètre est inférieur ou égal à l'épaisseur du joint. Celui-ci est apte à venir en butée par sa face inférieure contre une paroi d'appui, fixe, quand la tige creuse est enfoncée, de sorte que le joint est alors comprimé et dégage au moins partiellement l'orifice d' admission, afin de permettre un écoulement de produit en direction de ladite au moins une arrivée de produit. Le joint peut en outre, par exemple, s'appliquer à sa périphérie contre la surface intérieure d'un conduit dans lequel la tige creuse peut se déplacer axialement, ce conduit pouvant communiquer librement avec l'extérieur, auquel cas le joint permet d'obtenir une fermeture étanche de l'intervalle situé entre la tige creuse et la surface intérieure du conduit. Ce conduit peut se raccorder à son extrémité supérieure à un couvercle fermant supérieurement le récipient contenant le produit, par exemple. La tige creuse peut être actionnée par exemple par le déplacement d'un bouton-poussoir commandant l'émission de gaz vecteur. L'espace intérieur à la paroi d'appui contre laquelle le joint peut venir en butée par sa face inférieure peut communiquer avec le récipient contenant le produit, par exemple par l'intermédiaire d'une gorge annulaire réalisée dans une paroi de fond du récipient. Le conduit précité peut se raccorder de manière étanche à la paroi d'appui tubulaire précitée.

Selon un aspect de l'invention, le dispositif peut comporter un bouton-poussoir réalisé par assemblage d'une partie inférieure et d'une partie supérieure. La partie inférieure peut être réalisée par exemple d'un seul tenant avec une tige d'actionnement, et s'étendre dans un passage, par exemple central, du récipient contenant le produit. La tige de commande de la valve contenant le gaz vecteur peut être engagée dans la tige d'actionnement du bouton-poussoir.

La réserve de produit peut comporter initialement entre 5 et 200 cm³ de produit, par exemple, voire entre 10 et 100 cm³.

Le gaz peut être présent au moins sous une phase liquide à l'intérieur du récipient contenant le gaz vecteur.

D'autres caractéristiques et avantages de la présente invention ressortiront à la lecture d'exemples non limitatifs de mise en oeuvre de l'invention, et à l'examen du dessin annexé, sur lequel :
- la figure 1 est une vue schématique en perspective d'un dispositif de pulvérisation conforme à un premier exemple de mise en oeuvre de l'invention,
- la figure 2 est une vue schématique et partielle de dessus de la tête de pulvérisation de la figure 1,
- les figures 3 et 4 sont des coupes partielles illustrant deux variantes de réalisation de la tête de pulvérisation,
- la figure 5 est une vue schématique avec coupe partielle d'un dispositif de pulvérisation conforme à un deuxième exemple de mise en oeuvre de l'invention.
- la figure 6 est une vue schématique avec coupe axiale partielle d'un dispositif de pulvérisation conforme à un troisième exemple de mise en oeuvre de l'invention,
- la figure 7 est une vue de dessus du dispositif de la figure 6,
- la figure 8 est une vue analogue à la figure 6, représentant le dispositif lors de la pulvérisation du produit,
- la figure 9 est une coupe axiale schématique représentant un dispositif conforme à un quatrième exemple de mise en oeuvre de l'invention,
- les figures 10 et 11 sont des coupes axiales schématiques représentant une valve à trois positions,
- la figure 12 est une section axiale, schématique et partielle, selon XII-XII de la figure 13, d'un autre exemple de dispositif de pulvérisation,
- la figure 13 est une vue de dessus, partielle et schématique, selon la flèche XIII de la figure 12,
- la figure 14 est une section axiale, schématique et partielle, selon XIV-XIV de la figure 13,
- la figure 15 est une vue schématique, en perspective éclatée, partielle, du dispositif de la figure 12,
- la figure 16 est une vue à échelle agrandie du détail XVI de la figure 12,
- les figures 17 et 18 représentent schématiquement d'autres exemples d'obturateurs,
- la figure 19 représente de manière schématique un exemple de micro fuite, et,
- la figure 20 représente de manière schématique un exemple de clapet de reprise d'air.

Dans toute la description qui suit, y compris dans les revendications, les expressions « comportant un » ou « comprenant un » doivent être considérées comme étant synonymes de « comportant au moins un ».

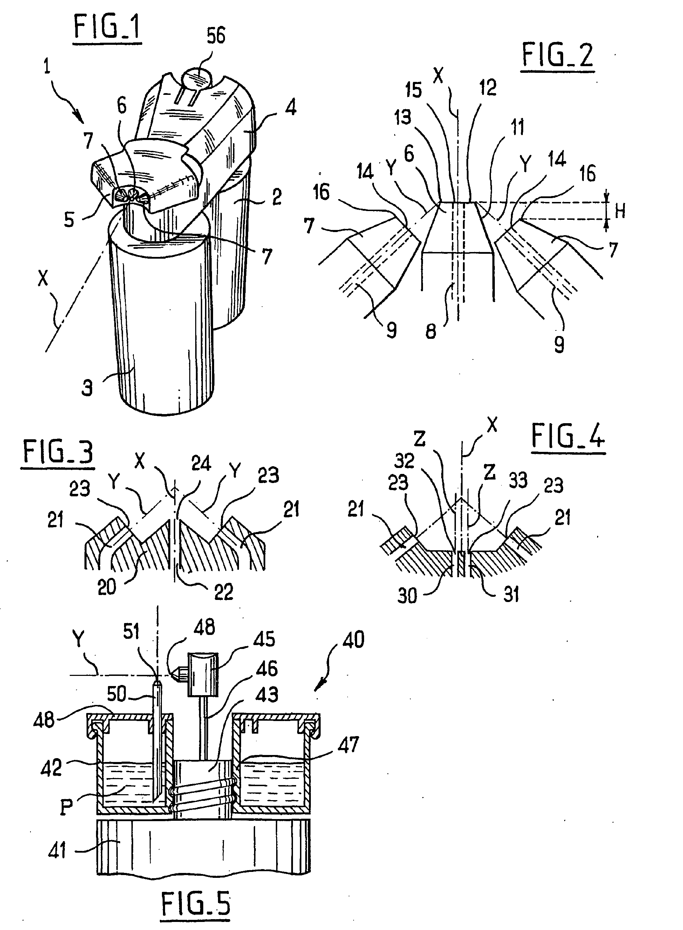

On a représenté sur la figure 1 un dispositif de pulvérisation 1 conforme à un premier exemple de mise en oeuvre de l'invention, comportant un premier récipient 2 contenant une réserve de gaz vecteur et un deuxième récipient 3 contenant un liquide à pulvériser.

Dans l'exemple considéré, le gaz vecteur est présent à l'état liquéfié dans le récipient 2 et sa nature est choisie de manière à être compatible avec une pulvérisation sur un support kératinique tel que la peau, les ongles ou les cheveux.

Le gaz vecteur est par exemple du gaz comprimé, notamment de l'air, du butane, de l'isobutane, de l'isopropane ou un composé fluoré.

Le gaz vecteur peut être présent à l'état liquéfié.

Bien entendu, on ne sort pas du cadre de la présente invention lorsque d'autres gaz vecteurs sont utilisés, par exemple de l'azote comprimé.

Le produit contenu dans le récipient comporte au moins 0,3 % en poids de particules solides, conformément à l'invention, et au moins un dispersant ou un gélifiant.

On se reportera utilement au début de la description où sont donnés de nombreux exemples de composés pouvant être présents dans le produit.

Le dispositif de pulvérisation 1 comporte, outre les récipients 2 et 3, un ensemble de distribution 4 comportant une tête de pulvérisation 5 et un organe d'actionnement 56 tel qu'un bouton-poussoir, actionnant une valve de commande non apparente sur le dessin.

La tête de pulvérisation 5 comporte une buse centrale 6 pour la distribution du liquide contenu dans le récipient 3 et deux buses latérales 7 pour l'émission du gaz vecteur.

On a représenté plus en détail sur la figure 2 les buses 6 et 7.

La buse 6 présente un canal intérieur 8, d'axe X, ce dernier coïncidant avec l'axe de pulvérisation.

Les buses 7 comportent chacune un canal intérieur 9 d'axe Y, les axes Y faisant chacun un angle de 45° avec l'axe X et étant contenus dans le même plan que l'axe X.

Le canal 8 communique en permanence avec le produit contenu dans le récipient 3, étant prolongé à l'intérieur du récipient par un tube plongeur, et les canaux 9 communiquent avec le gaz vecteur contenu dans le récipient 2 par l'intermédiaire de la valve actionnée par le bouton-poussoir 56.

Le diamètre des orifices 14 de sortie du gaz vecteur est de 0,4 mm par exemple et celui de l'orifice 15 de sortie du liquide de 0,9 mm, par exemple.

Lorsque l'utilisateur appuie sur le bouton-poussoir 56, du gaz vecteur circule dans les canaux 9 et se détend à la sortie des buses 7, provoquant par effet Venturi une dépression en avant de la buse 6 et l'aspiration de produit dans le canal 8.

Du fait de l'orientation des axes Y, les jets de gaz vecteur émis par les buses 7 se rencontrent, ce qui permet d'obtenir une pulvérisation ayant des caractéristiques de granulométrie et de géométrie de spray compatibles avec l'obtention de résultats de maquillage satisfaisants.

Dans la configuration particulière illustrée sur la figure 2, la buse 6 agit en outre comme un élément formant déflecteur et dévie vers l'avant une partie des jets de gaz vecteur émis par les buses 7.

En d'autres termes, les jets de gaz vecteur heurtent au moins partiellement la buse 6 et prennent alors une orientation plus proche de celle de l'axe de pulvérisation.

On remarquera que la buse 6 présente une partie tronconique creuse 11 dont la génératrice fait un angle avec l'axe de pulvérisation X qui est inférieur à l'angle que fait chaque axe Y avec l'axe X.

Dans l'exemple représenté sur la figure 2, la buse 6 présente une face d'extrémité 12 plane et perpendiculaire à l'axe X et l'axe Y de chaque buse 7 intercepte le bord 13 circulaire de la face d'extrémité 12.

La distance H entre la face d'extrémité 12 de la buse 6 et le bord 16 de la face avant de chaque buse 7 est de 1,7 mm environ, par exemple.

Dans l'exemple de réalisation des figures 1 et 2, les buses 6 et 7 sont constituées par des pièces rapportées.

Bien entendu, on ne sort pas du cadre de la présente invention lorsque les orifices de sortie du produit et du gaz vecteur sont constitués par les extrémités de canaux intérieurs réalisés dans une pièce monobloc, comme illustré sur la figure 3.

Sur cette figure, on a représenté une tête de pulvérisation 20 traversée par des canaux latéraux 21 communiquant avec la réserve de gaz vecteur et un canal central 22 communiquant avec la réserve de liquide et débouchant à l'extérieur par un orifice 24 d'axe X.

Les canaux 21 débouchent à l'extérieur par des orifices distincts 23, d'axes Y sécants avec l'axe X.

Dans le mode de réalisation de la figure 3, les jets de gaz vecteur émis par les canaux 21 se percutent directement, sans déflexion préalable par un élément formant déflecteur tel que la buse 6 précédemment décrite.

L'invention n'est pas limitée à la distribution d'un liquide unique et l'on peut, sans sortir du cadre de la présente invention, distribuer un mélange d'au moins deux produits, l'un des produits pouvant par exemple être contenu dans le récipient contenant le gaz propulseur et entraîné avec ce dernier.

Les deux produits peuvent également être contenus dans deux récipients distincts, différents de celui contenant le gaz vecteur.

Dans ce cas, l'amenée des liquides peut se faire par deux voies séparées, comme illustré sur la figure 4.

Sur cette figure, le canal 22 a été remplacé par deux canaux 30 et 31 communiquant respectivement avec deux réserves de produits différents, par exemple deux produits devant être conditionnés séparément et mélangés extemporanément.

Les axes Z des orifices 32 et 33 des canaux 30 et 31 sont parallèles à l'axe X de pulvérisation.

L'axe de pulvérisation X peut être horizontal ou avoir une orientation autre, en fonction de l'ergonomie du dispositif de pulvérisation et de l'emplacement de la zone à traiter.

Le dispositif de pulvérisation peut être prévu pour pouvoir fonctionner tête en haut ou tête en bas.

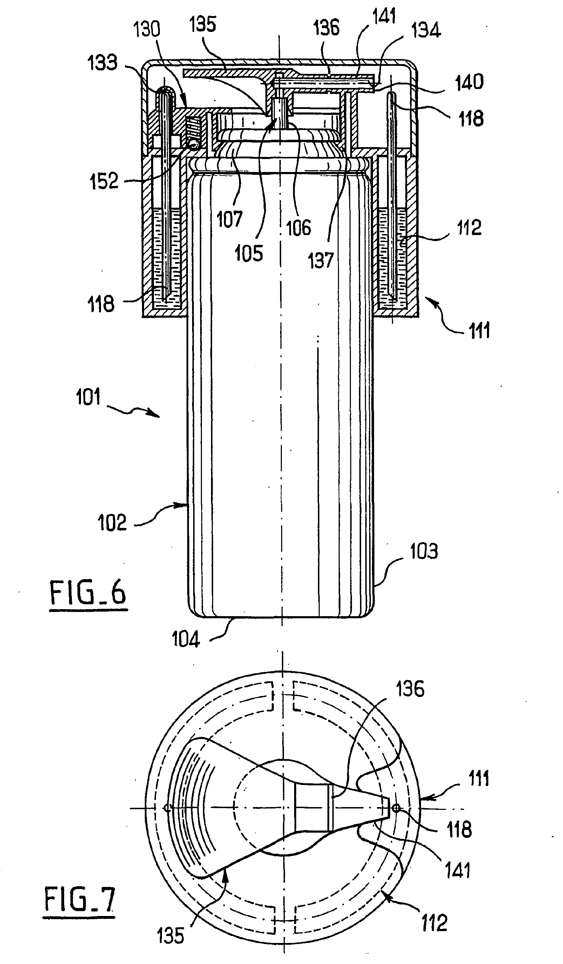

Le produit peut être contenu dans un récipient amovible, comme cela va maintenant être décrit en référence à la figure 5.

Sur cette figure, on a représenté un dispositif 40 comportant un récipient 41 contenant du gaz vecteur et un récipient 42 contenant une réserve de produit P.

Ce produit P comporte des particules solides et au moins un gélifiant ou un dispersant conformément à l'invention.

Le récipient 41 est pourvu d'un col fileté 43.

Une tête de pulvérisation 45 est fixée sur la tige creuse de commande 46 de la valve du récipient 41.

Le récipient 42 comporte une jupe de montage annulaire 47, agencée pour se visser sur le col fileté 43.

Le récipient 42 est fermé de manière étanche en partie supérieure par un couvercle 48.

Le bouton-poussoir 45 présente deux orifices 48 de distribution de gaz vecteur, formant un angle entre eux et dont les axes Y sont sécants et se coupent sensiblement à l'aplomb d'un organe d'amenée de liquide 50, constitué dans l'exemple de la figure 5 par un tube plongeant jusque dans le fond du récipient 42.

Le tube 50 traverse de manière étanche le couvercle 48.

L'extrémité supérieure du tube 50 est pourvue d'un orifice 51 permettant la sortie du liquide sous l'effet de la dépression créée par l'éjection de gaz vecteur par les orifices 48, lorsque l'utilisateur appuie sur le bouton-poussoir 45.

La fixation amovible du récipient 42 sur le récipient 41 permet à l'utilisateur de reconstituer la réserve de liquide lorsque celle-ci est épuisée en remplaçant le récipient 42 par un nouveau récipient rempli de produit.

L'utilisateur peut également, grâce au montage amovible du récipient 42 sur le récipient 41, sélectionner le récipient 42 parmi plusieurs récipients contenant des produits différents, par exemple des produits de couleurs différentes.

Ainsi, l'utilisateur n'utilise qu'un seul récipient 41 contenant le gaz vecteur en liaison avec un récipient choisi parmi plusieurs récipients contenant des produits différents.

Il convient de remarquer que le récipient 42 ne comporte pas d'orifice de reprise d'air autre que le canal intérieur du tube 50.

Lorsque du produit est distribué lors de l'actionnement du bouton-poussoir 45, une dépression se créée dans le récipient 42 et lors de l'arrêt de la distribution, le retour à la pression d'équilibre dans le récipient 42 s'accompagne d'une descente du produit dans le tube 50, ce qui permet de réaliser en quelque sorte un auto-nettoyage du tube 50 et limite le risque de bouchage de celui-ci.

Le dispositif 101 représenté aux figures 6 à 8 est décrit de manière détaillée dans la demande de brevet français FR-A-2 781 208.

Ce dispositif 101 comprend un récipient 102 contenant un gaz pressurisé, notamment de l'air. Le récipient 102 est constitué d'un bidon comprenant un corps 103 dont une extrémité est fermée par un fond 104. Sur l'extrémité opposée au fond est montée une valve 105 comportant une tige émergente 106, du type à actionnement par enfoncement. La valve 105 est portée par une coupelle 107 fixée sur le récipient 102 par sertissage. La valve 105 est elle-même montée sur la coupelle 107 par sertissage.

Sur le récipient 102, est monté libre en rotation un organe porte-réservoirs 111, de forme annulaire, définissant deux réservoirs 112 contenant chacun un produit cosmétique à pulvériser.

Un conduit 118 plonge dans chaque réservoir.

Une frette 130 est accrochée par encliquetage sur le récipient 102.

Cette frette 130 est immobilisée en rotation, notamment par serrage, par rapport au récipient, et immobilise axialement le porte-réservoirs 111.

La frette 130 comporte une gorge annulaire 133 apte à recouvrir les conduits 118 des réservoirs 112, de manière à les isoler de l'extérieur.

Un canal 134 de distribution de gaz vecteur est formé à l'intérieur d'une avancée 141 et débouche latéralement par un orifice de sortie 140.

Le canal 134 est relié à un bouton-poussoir 135 par l'intermédiaire d'une charnière film 136 orientée perpendiculairement à l'axe du récipient 102.

L'orifice de sortie 140 est disposé de manière à pouvoir être situé le plus près possible de l'extrémité supérieure d'un conduit 118. Cette distance est par exemple de l'ordre de 1 mm.

L'avancée 141 est directement encliquetée sur le récipient 102 par l'intermédiaire d'une patte d'accrochage 137.

Le positionnement précis du porte-réservoirs 111 par rapport à l'orifice de sortie de gaz vecteur 140 se fait grâce à un mécanisme d'indexation à bille 152 et ressort, autorisant autant de positions indexées qu'il existe de réservoirs 112.

Avantageusement, le mécanisme de positionnement indexé permet quatre positions indexées, espacées de 90° : deux premières positions espacées de 180° dans lesquelles l'orifice de sortie 140 est en regard de l'un ou l'autre des conduits 118 et deux positions intermédiaires de stockage dans lesquelles l'orifice de sortie 140 n'est en regard d'aucun des conduits 118, les deux positions intermédiaires étant à 90° par rapport aux deux premières positions.

Chaque conduit 118 a une extrémité inférieure 117 trempant dans le fond du réservoir 112 correspondant, l'autre extrémité émergeant hors de ce réservoir 112.

Le canal 134 est orienté sensiblement perpendiculairement à l'axe des conduits 118.

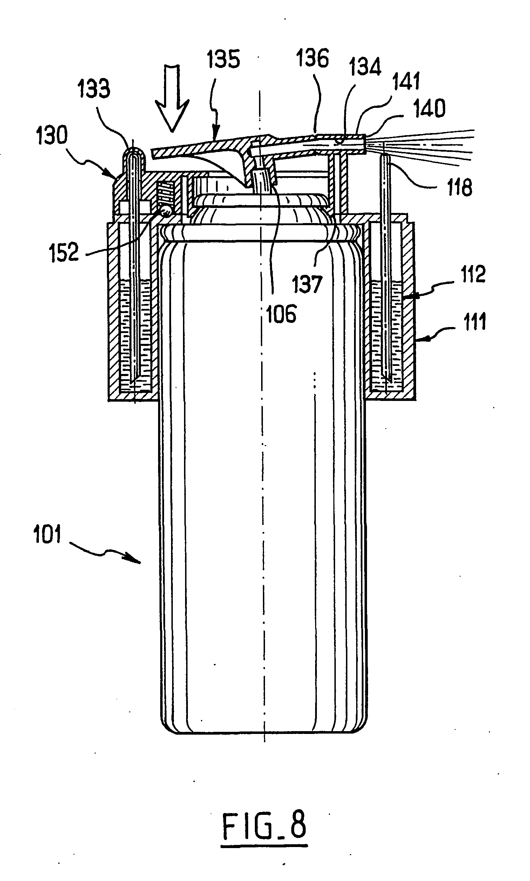

Le produit contenu dans le réservoir 112 sélectionné et dont le conduit 118 se situe au voisinage de l'orifice 140 est pulvérisé lorsque l'utilisateur appuie sur le bouton-poussoir 135, comme illustré à la figure 8.

Dans les exemples décrits en référence aux figures 1 à 8, le produit est pulvérisé grâce à la dépression créée par effet Venturi par le gaz vecteur.

Des essais concluants ont été réalisés avec le dispositif des figures 1 et 2, avec un fond de teint non huileux ayant la composition suivante :

| BY 29-119 | |
|---|---|
| (silicone réticulé en dispersion aqueuse de Dow Corning Toray) | 40 % |
| Pigments (oxyde de fer et dioxyde de titane) (particules solides) | 7 % |
| Propylène glycol | 7 % |
| Glycérine | 4 % |
| Carboxymethyl cellulose (gélifiant) | 0,1 % |
| Poudre d'amidon (particules solides) | 2,5 % |
| Talc modifié hydrophile (particules solides) | 2,5 % |
| Conservateur | q.s. |
| Eau | q.s.p. 100 % |

Pour préparer ce fond de teint, on disperse les pigments dans le propylène glycol, la glycérine, et on réalise un gel avec la carboxymethyl cellulose, l'eau et le conservateur.

On agite le gel de manière modérée à froid (environ 25° C) pendant dix minutes.

On y disperse les particules solides, à savoir le composé BY 29-119, le talc et l'amidon toujours à froid et on agite l'ensemble pendant dix minutes.

On obtient un fond de teint très fluide, stable au cours du temps dans les conditions habituelles de conservation, notamment pendant deux mois à 45° C.

La viscosité est de 50 centipoises, mesurée dans les conditions décrites précédemment.

Aucun bouchage de la buse centrale 6 n'a été constaté en cours de fonctionnement.

Un autre exemple de composition, pour le maquillage du corps, est le suivant:

| | |
|---|---|
| Pigments (particules solides) | 7 % |
| Nacres (particules solides) | 3 % |
| Propylène glycol | 2,65 % |
| Xanthane (gélifiant de type polysaccharide) | 0,5 % |
| Dispersant à pigments : ARLATONE 3315 commercialisé par la société UNIQEMA (acrylate copolymer et propylène glycol à 40 % dans un mélange eau et propylène (50/50), et sel de sodium.) | 0,18 % |
| BYK-019 Diméthicone copolyol vendu par la société BYK-CERA dans le dipropylène glycol monométhyl ether (anti-mousse) | 0,06 % |
| Eau | qsq 100 % |

Bien entendu, l'invention n'est pas limitée aux exemples de réalisation décrits.

En particulier, bien que l'utilisation dans le produit contenant des particules solides d'un gélifiant soit particulièrement avantageuse lorsque le produit est pulvérisé au moyen de l'un des dispositifs portatifs autonomes décrits en référence aux figures 1 à 8, la présence d'un gélifiant dans le produit s'avère également avantageuse lorsque le produit est pulvérisé en étant envoyé sous pression dans une buse, le produit étant éventuellement mélangé à un gaz propulseur.

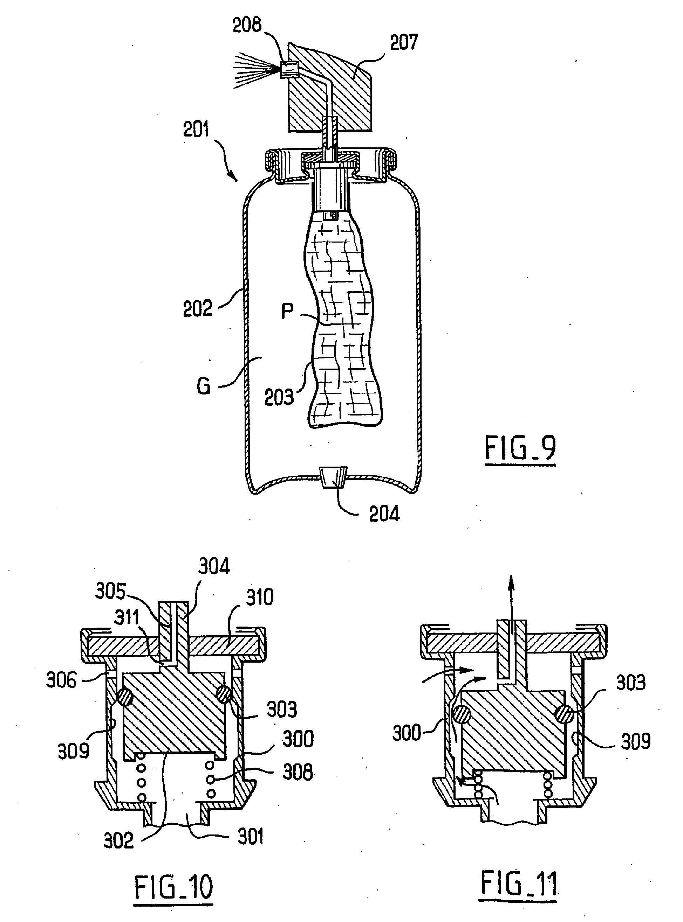

A titre d'exemple, on a représenté sur la figure 9 un dispositif 201 comportant un récipient 202 du type bidon aérosol, comprenant une poche 203 contenant le produit P à distribuer, l'espace à l'extérieur de la poche 203 dans le récipient 202 étant rempli d'un gaz G, par exemple de l'air comprimé.

Le fond du bidon aérosol 202 est équipé d'une valve 204 permettant de le recharger en air comprimé et le récipient 202 comporte en partie supérieure une valve 205 permettant de distribuer le produit P contenu dans la poche 203.

Cette valve 205 comporte une tige de commande sur laquelle est monté un bouton-poussoir 207 portant une buse 208.

La buse 208 peut présenter de multiples configurations et n'a été représentée que schématiquement, dans un souci de clarté du dessin.

Lorsque l'utilisateur appuie sur le bouton-poussoir 207 et provoque l'enfoncement de la tige de commande, le produit P contenu dans la poche 203 est envoyé sous pression dans la buse 208, en étant de préférence mélangé à du gaz.

On ne sort pas du cadre de la présente invention lorsque l'on pulvérise le produit sans le mélanger au gaz.

Lorsque le produit est pulvérisé en étant mélangé au gaz propulseur, on peut utiliser, par exemple, une valve à au moins trois positions, telle que représentée schématiquement sur les figures 10 et 11, une telle valve étant décrite de manière plus détaillée dans la demande de brevet européen EP-0 709 305.

Cette valve comporte un corps 300, communiquant avec la réserve de produit à son extrémité inférieure par un orifice 301.

Un organe 302 comportant un joint d'étanchéité 303 est mobile dans le corps 300 contre l'action d'un ressort 308.

L'organe 302 est réalisé d'un seul tenant avec la tige 304 de commande de la valve, celle-ci étant pourvue d'un canal 305 pour la sortie du produit ou du gaz propulseur.

Le canal 305 débouche inférieurement par un orifice latéral 311, lequel est obturé par un joint 310 lorsque la valve est au repos.

Le corps 300 est pourvu d'un orifice 306 de prise de gaz propulseur en partie supérieure.

La poche contenant le produit est fixée sur le corps 300 sans boucher l'orifice 306.

L'organe 302 porte un joint torique d'étanchéité 303. Une gorge annulaire 309 est formée sur la surface intérieure du corps 300.

Au repos, le joint torique 303 s'applique sur la surface intérieure du corps 300 au-dessus de la gorge annulaire 309, de sorte que le conduit 305 ne communique avec l'intérieur du récipient que par l'orifice 306 de prise de gaz.

Lorsque la tige de commande 304 est enfoncée sur une première partie de sa course vers le bas, le joint torique 303 continue à s'appliquer de manière étanche sur la surface intérieure du corps 300 tandis que l'orifice 311 débouche sous le joint 310, ce qui permet seulement un passage de gaz propulseur par l'orifice 306, comme illustré à la figure 10.

Lorsque la tige de commande 304 est davantage enfoncée, le joint torique 303 atteint la gorge 309 et cesse de s'appliquer sur la surface intérieure du corps 300, ce qui permet un passage simultané de produit de gaz propulseur, comme illustré à la figure 11.

La position de la figure 10 permet de purger le produit contenu dans le circuit de distribution avant le retour de la valve à sa position de repos.

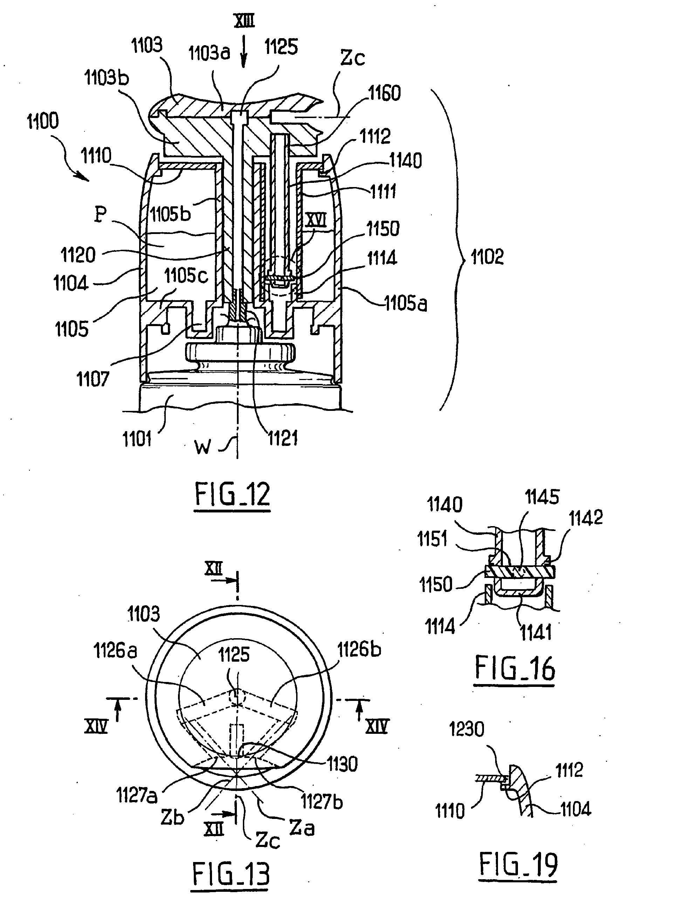

Le dispositif de pulvérisation 1100 représenté à la figure 12 comporte un récipient pressurisé 1101 contenant par exemple un gaz vecteur sous forme liquéfiée, par exemple du butane, de l'isopropane, de l'isobutane ou un composé fluoré, et un ensemble de distribution 1102 comportant un bouton-poussoir 1103 et un récipient 1104 contenant un produit P tel que décrit précédemment.

Le récipient 1104 comporte, dans l'exemple décrit, une cavité 1105 de forme générale annulaire autour d'un axe W, cette cavité 1105 contenant le produit P étant délimitée radialement à l'extérieur par une première paroi tubulaire 1105a et radialement à l'intérieur par une deuxième paroi tubulaire 1105b. Les parois 1105a et 1105b sont réunies à leur partie inférieure par une paroi de fond 1105c, qui comporte une gorge annulaire 1107, d'axe W, dont le rôle sera précisé plus loin.

La cavité 1105 est fermée supérieurement par un couvercle 1110, sur la face inférieure duquel se raccorde un conduit 1111 d'axe parallèle à l'axe W. Le couvercle 1110 vient prendre appui contre un épaulement 1112 réalisé à l'extrémité supérieure de la paroi 1105a.

Le conduit 1111 s'étend sur sensiblement toute la hauteur de la cavité 1105 et vient s'assembler à son extrémité inférieure avec une paroi 1114, formant saillie vers le haut sur la paroi de fond 1105c, à l'aplomb de la gorge 1107. L'assemblage du conduit 1111 et de la paroi 1114 est réalisé par exemple par emmanchement. La paroi 1114 précitée peut être continue ou discontinue et peut par exemple former des plots.

Le conduit 1111 et le couvercle 1110 peuvent être réalisés d'un seul tenant, par exemple.

Le bouton-poussoir 1103 est formé par exemple par l'assemblage d'une partie supérieure 1103a et d'une partie inférieure 1103b. Dans l'exemple illustré, cette dernière est réalisée d'un seul tenant avec une tige creuse 1120 d'actionnement d'une valve du récipient pressurisé 101, pouvant coulisser à l'intérieur de la paroi 1105b.

Cette valve peut comporter une tige creuse 1121, engagée de manière étanche dans l'extrémité inférieure de la tige 1120, et venant en appui par un épaulement contre la face d'extrémité inférieure de la tige 1120.

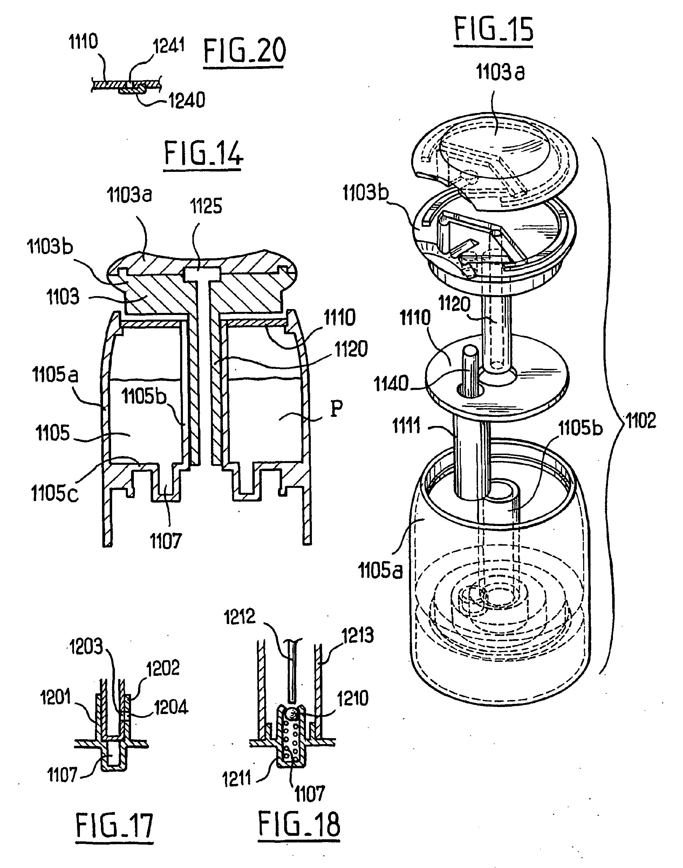

Le bouton-poussoir 1103 peut être déplacé selon l'axe W pour agir sur la tige de commande 1121, auquel cas du gaz vecteur circule dans le conduit intérieur de la tige 1120 et gagne une cavité 1125, avec laquelle communiquent, comme on peut le voir sur la figure 14, deux conduits intérieurs 1126a et 1126b débouchant à l'extérieur du bouton-poussoir par des orifices de sortie de gaz vecteur 1127a et 1127b. Les orifices 1127a et 1127b ont par exemple des axes Za et Zb sensiblement perpendiculaires et font chacun par exemple un angle de 45° environ avec la direction de pulvérisation.

Le bouton-poussoir 1103 comporte également un orifice 1130 d'arrivée du produit, d'axe Zc, par exemple confondu avec la direction de pulvérisation. L'orifice 1130 communique par exemple avec l'intérieur d'une tige creuse 1140 fermée à son extrémité inférieure par une paroi 1141, comme on peut le voir sur la figure 16, et sur laquelle est engagé un joint annulaire 1150 servant d'obturateur, ce joint 1150 étant par exemple réalisé en élastomère.

Dans l'exemple illustré, le joint 1150 vient en appui par sa face supérieure plane 1151 contre une nervure annulaire 1142 de la tige 1140. Cette dernière présente au moins un orifice 1145 d'admission de produit, dont le diamètre est inférieur ou égal à l'épaisseur nominale du joint 1150, mesurée le long de l'axe de la tige 1140. L'orifice 1145 est positionné de telle sorte que le joint 1150, lorsqu'il est en appui au repos contre la nervure 1142, recouvre complètement l'orifice 1145 et empêche le produit P contenu dans la cavité 1105 de pénétrer par l'orifice 1145 dans la tige 1140.

La tige 1140 est par exemple fixée à son extrémité supérieure dans un logement 1160 du bouton-poussoir 1103 et peut se déplacer conjointement avec la tige 1120 lorsque l'utilisateur appuie sur le bouton-poussoir 1103. Lors de l'enfoncement de la tige 1140, le joint 1150 est comprimé entre la paroi 1114 et la nervure 1142, une telle compression ayant pour effet de réduire son épaisseur et de libérer au moins partiellement l'orifice 1145, de telle sorte que du produit contenu dans la cavité 1105 peut circuler à travers l'orifice 1145, remonter dans la tige 1140 et gagner l'orifice d'arrivée de produit 1130.

Lorsque le bouton-poussoir 1103 est relâché, la tige 1140 remonte avec ce dernier et le joint 1150 peut reprendre grâce à une mémoire de forme une épaisseur suffisante pour obturer l'orifice 1145.

Le fonctionnement du dispositif 1100 est le suivant. Pour pulvériser du produit P, l'utilisateur appuie sur le bouton-poussoir 1103, ce qui provoque l'enfoncement de la tige 1121 de la valve du récipient et l'émission de gaz vecteur dans le canal intérieur de la tige 1120. Le gaz vecteur peut circuler par les conduits 1126a et 1126b et sortir par les orifices 1127a et 1127b, ce qui crée par effet Venturi en avant de l'orifice d'arrivée de produit 1130 une dépression. L'enfoncement du bouton-poussoir 1103 a également pour conséquence de déplacer la tige 1140 et de comprimer le joint 1150, comme expliqué plus haut. L'orifice 1145 est alors au moins partiellement libéré et du produit P contenu dans la cavité 1105 peut, sous l'effet de la dépression précitée, remonter dans le canal intérieur de la tige 1140 et gagner l'orifice 1130. Le produit est alors pulvérisé dans la direction de l'axe Zc, tant que l'utilisateur appuie sur le bouton-poussoir 1103. Lorsque ce dernier est relâché, il peut remonter dans sa position de repos du fait que la tige de commande 1121 est rappelée par des moyens élastiques propres au récipient 1101 dans sa position initiale et sous l'effet de l'élasticité propre du joint 1150. Ce dernier, en reprenant sa forme initiale, obture l'orifice 1145, de sorte qu'en cas de transport du dispositif 1100 en position allongée ou à l'envers, le produit P reste contenu dans la cavité 1105 et ne risque pas de fuir par l'orifice d'arrivée de produit 1130.

Le récipient 1104 peut être par exemple fixé de manière amovible sur le récipient contenant le gaz vecteur, de manière à permettre, le cas échéant, de changer de récipient 1104 parce que la réserve de produit est épuisée. Cela peut permettre aussi de remplacer le récipient 1101 ou de pulvériser différents produits successivement au moyen d'un unique récipient 1101.

Dans l'exemple correspondant aux figures 12 à 17, l'arrivée de produit est constituée par l'extrémité d'un conduit formé dans le bouton-poussoir mais on ne sort pas du cadre de la présente invention lorsque l'arrivée de produit comporte un matériau capable d'absorber par capillarité du produit, par exemple une mèche, un feutre ou un fritté.

On peut également utiliser une pluralité de réserves de produits différentes, montées sur un barillet, de manière similaire à ce qui a été décrit dans la demande de brevet français FR-A-2 781 208.

Bien entendu, l'invention n'est pas limitée aux exemples qui viennent d'être décrits et, d'une manière générale, on peut remplacer l'obturateur qui a été décrit par tous moyens d'obturation appropriés, par exemple des moyens d'obturation comportant, comme illustré à la figure 17, deux parois coaxiales 1201, 1202 mobiles l'une par rapport à l'autre, constituant un obturateur.

La paroi 1202, la plus intérieure, peut être formée par exemple par une tige creuse solidaire du bouton-poussoir, étant fermée à son extrémité inférieure. Le produit peut remonter à l'intérieur de cette tige lorsqu'une dépression est créée par une émission de gaz vecteur.

La paroi 1201, la plus extérieure, est fixe et elle est apte à obturer un orifice 1203 de la paroi 1202 lorsque le bouton-poussoir est au repos. La paroi 1202 comporte un orifice 1204 en regard duquel l'orifice 1203 peut venir, lorsque le bouton-poussoir est enfoncé, ce qui libère au moins partiellement l'orifice 1203, pour permettre au produit de gagner l'arrivée de produit.

Un clapet, par exemple à bille, commandé en ouverture par le déplacement du bouton-poussoir pourrait également être utilisé, comme illustré à la figure 18. Un tel clapet peut prendre une position fermée lorsque le bouton-poussoir est relâché par l'utilisateur. Dans l'exemple illustré, le clapet comporte une bille 1210 formant obturateur, rappelée dans une position d'obturation par un ressort 1211. Une tige d'actionnement 1212 est agencée pour être déplacée vers le bas lorsque le bouton-poussoir est enfoncé. Le produit peut alors s'écouler par un conduit 1213 pour gagner la zone où la dépression est créée, afin d'être pulvérisé.

La gorge annulaire 1107 pourrait, dans des variantes de réalisation, être supprimée, par exemple si l'on permet au produit de gagner l'espace intérieur à la paroi 1114, par exemple grâce à des ouvertures réalisées dans cette paroi et dans l'extrémité inférieure du conduit 1111.

On ne sort pas du cadre de l'invention lorsque la dépression est créée par l'émission, par un seul orifice, du gaz vecteur.

La tige de commande 1121 du récipient contenant le gaz vecteur pourrait encore commander la distribution de gaz vecteur en étant inclinée par rapport à l'axe W, à condition de réaliser le bouton-poussoir en conséquence.

Le récipient 1104 peut être réalisé sans reprise d'air ou avec reprise d'air, notamment si une utilisation prolongée est envisagée.

Pour permettre une reprise d'air, on peut par exemple réaliser une micro fuite 1230, comme illustré sur la figure 19, à travers le couvercle 1110 ou entre le couvercle et la paroi 1105a du récipient 1104, ce qui correspond à l'exemple représenté sur la figure 20. Une telle microfuite est réalisée de manière à empêcher le passage du produit notamment lorsque le dispositif est en position couchée, mais à permettre le passage de l'air.

On peut encore prévoir, par exemple, un clapet 1240 apte à obturer un orifice de reprise d'air 1241, comme illustré à la figure 21. Le clapet 1240 s'ouvre en cas de dépression à l'intérieur du récipient contenant le produit et obture l'orifice 1241 dans le cas contraire.

Le clapet 1240 peut être par exemple surmoulé sur le couvercle 1110, mais pourrait encore être réalisé autrement sans que l'on sorte du cadre de la présente invention.

## Revendications

1. Dispositif autonome de construction unitaire (1 ; 101 ; 201;1100) pour pulvériser un produit cosmétique sur un support kératinique, notamment la peau, comportant une réserve de produit à pulvériser et un récipient contenant un gaz, ainsi qu'une valve permettant, lorsqu'elle est actionnée, la pulvérisation du produit, ce dernier contenant des particules solides et au moins un gélifiant, dispositif dans lequel la réserve de produit est contenue dans un récipient différent de celui contenant le gaz ou le produit est contenu dans une poche souple placée à l'intérieur du récipient contenant le gaz.

2. Dispositif selon la revendication 1, **caractérisé par le fait que** le gélifiant est choisi de manière à ne pas cristalliser dans le milieu qui le contient à température ambiante.

3. Dispositif selon la revendication 2, **caractérisé par le fait que** le gélifiant est choisi en dehors de la liste suivante :
- les cires, notamment les cires hydrocarbonées ou les cires de silicone (alkyle ou alcoxydiméticone).
- les esters d'alcool gras et d'acide gras, dont les cérides et les stérides ou les esters d'acides gras et de glycérophoscholine, tels que les phospholipides,
- les acides gras et aminés notamment les céramides.

4. Dispositif selon l'une des revendications précédentes, **caractérisé par le fait que** le produit comporte plus de 0,3 % en poids de particules solides, de préférence entre 5 et 70 % en poids de particules solides, de préférence entre 20 et 70 %, de préférence encore entre 30 et 60 % notamment lorsque le produit est un fond de teint, et **par le fait que** le gélifiant est dans une proportion comprise entre 0,01 et 10 % en poids, de préférence dans une proportion supérieure ou égale à 0,1 %.

5. Dispositif selon l'une des revendications précédentes, **caractérisé par le fait que** le produit comporte des pigments.

6. Dispositif selon la revendication précédente, **caractérisé par le fait que** les pigments sont choisis dans la liste suivante : l'oxyde de fer, de titane ou de zinc, les laques organiques, les nacres minérales, notamment de mica, de titane ou micatitane et leurs mélanges.

7. Dispositif selon l'une quelconque des revendications précédentes, **caractérisé par le fait que** le produit comporte des particules choisies dans la liste suivante : poudres organiques, notamment particules de polyamide, de polyacrylate, de méthacrylate de méthyle, de polyuréthane, de Polyéthylène, de polystyrène réticulé, ou leurs mélanges, poudres d'origine minérale telles que le talc, l'argile, la silice, le nitrure de bore ou leurs mélanges, poudres d'origine végétale telles que l'amidon, poudres d'origine animale telles que la poudre de coquillages, silicones sous forme de billes de résine méthylsesquioxane, poudres fluorées telles que poudres de PTFE et leurs mélanges.

8. Dispositif selon l'une quelconque des revendications 1 à 7, **caractérisé par le fait que** les particules sont de taille micrométrique (taille du grain élémentaire).

9. Dispositif selon l'une quelconque des revendications 1 à 7, **caractérisé par le fait que** les particules sont de taille nanométrique.

10. Dispositif selon l'une quelconque des revendications précédentes, **caractérisé par le fait que** le ou les gélifiant(s) utilisé(s) sont hydrophiles.

11. Dispositif selon l'une quelconque des revendications 1 à 9, **caractérisé par le fait que** le ou les gélifiant(s) utilisé(s) sont lipophiles.

12. Dispositif selon la revendication 10, **caractérisé par le fait que** le produit comporte au moins un gélifiant hydrophile choisi parmi : les gélifiants hydrophiles de nature polymérique tels que les gélifiants de type polysaccharide, notamment la gomme de xanthane, de gellane, de guar ou ses dérivés, la cellulose et ses dérivés, les gélifiants de type vinylique, carboxyvinylique, acrylique, le polyuréthane, les gélifiants de nature non polymérique de type minéral comme les argiles, notamment le Veegum®, la Bentone® modifiée ou non ou les silices hydrophiles, ou leurs mélanges.

13. Dispositif selon la revendication 11, **caractérisé par le fait que** le produit comporte un gélifiant lipophile choisi parmi les gélifiants lipophiles suivants : les gélifiants de type polymère ou de type "organo gélateur", les polymères siliconés linéaires ou réticulés et leurs dérivés, les polymères dérivés des silicones, les silicones acryliques, les polycondensats de type polyamides, les polysaccharides. à chaînes hydrophobes ou des polyuréthanes ou leurs mélanges.

14. Dispositif selon l'une quelconque des revendications précédentes, **caractérisé par le fait que** le produit comporte des gélifiants minéraux pouvant être modifiés le cas échéant par des groupements organiques afin de gélifier des huiles. '

15. Dispositif selon les revendications 10 ou 11, **caractérisé par** la fait que le produit comporte et au moins un dispersant et au moins un gélifiant

16. Dispositif selon la revendication précédente, **caractérisé par le fait que** le produit comporte au moins un dispersant hydrophile choisi parmi les dispersants hydrophiles suivants : dispersants ayant au moins une charge anionique, notamment les dispersants carboxyliques, les sulfonates, les terpolymeres de l'acide acrylique, les polyaspartates, les dérivés de l'acide maléique ou leurs mélanges.

17. Dispositif selon la revendication 15, **caractérisé par le fait que** la produit comporte au moins un dispersant lipophile choisi parmi les dispersants lipophiles suivants : les carboxylates, les acryliques, les hydrocarbonés, avec cycle, notamment styrène, ou de type succinimide.

18. Dispositif selon l'une quelconque des revendications précédentes, **caractérisé par le fait que** la réserve de produit est contenue dans un récipient suffisamment clos pour permettre une conservation du produit dans le dispositif, en l'absence d'utilisation, supérieure à au moins un mois à température ambiante.

19. Dispositif selon l'une quelconque des revendications précédentes, **caractérisé par le fait que** la viscosité du produit est comprise entre 20 centipoises et 500 centipoises, la viscosité étant mesurée à l'aide d'un rhéomate RM 180 de la société RHEOMETRIC SCIENTIFIC, de type rotatif, utilisant un mobile adapté dit de type "1", "2" ou "3" selon la fluidité de la formule, après dix minutes d'un taux de cisaillement de 200/s, la température des mesures étant de 25°C.

20. Dispositif selon l'une quelconque des revendications précédentes, **caractérisé par le fait que** le produit comporte des actifs hydrophiles ou lipophiles tels que par exemple des agents bloqueurs d'UV (des filtres) ou des hydratants, une huile de soin, un anti-oxydant, des conservateurs ou des agents anti-mousse.

21. Dispositif selon l'une quelconque des revendications précédentes, **caractérisé par le fait que** la réserve de produit est contenue dans une cartouche amovible.

22. Dispositif selon l'une quelconque des revendications 1 à 20, **caractérisé par le fait que** la réserve de produit est contenue dans un récipient non amovible.

23. Dispositif selon l'une quelconque des revendications précédentes, **caractérisé par le fait qu'**il comporte un tube plongeant dans la réserve de produit

24. Dispositif selon l'une quelconque des revendications précédentes, **caractérisé par le fait que** la réserve de produit est contenue dans un récipient différent de celui contenant le gaz.

25. Dispositif selon l'une quelconque des revendications 1 à 11, **caractérisé par le fait que** le produit est contenu dans une poche souple placée à l'intérieur du récipient contenant le gaz.

26. Dispositif selon l'une quelconque des revendications précédentes, **caractérisé par** la fait que la valve est à au moins trois positions, à savoir une position de repos, une position permettant la distribution d'un mélange de produit et de gaz et une position permettant la distribution de gaz uniquement

27. Dispositif selon l'une quelconque des revendications 1 à 24, **caractérisé par le fait qu'**il comporte au moins un conduit (22) d'amenée de produit et au moins un conduit (21) d'amenée de gaz, de préférence deux, les conduits étant agencés de telle sorte que la sortie de gaz par le conduit d'amenée provoque une dépression apte à entraîner par aspiration du produit provenant du conduit d'amenée de produit

28. Dispositif selon l'une quelconque des revendications précédentes, **caractérisé par le fait que** le gaz est présent au moins sous une phase liquide à l'intérieur du récipient (2) contenant le gaz.

29. Dispositif selon la revendication 1, **caractérisé par le fait qu'**il comporte une réserve de gaz, au moins une arrivée de produit apte à être mise en communication fluidique avec une réserve dudit produit, ce dernier étant aspiré dans la réserve grâce à une dépression créée dans le voisinage de ladite au moins une arrivée de produit au moyen d'une émission dudit gaz, et au moins un obturateur (1150 ; 1201) apte à interrompre la communication fluidique entre ladite au moins une arrivée de produit et la réserve de produit.

30. Dispositif selon la revendication 29, **caractérisé par le fait que** l'arrivée de produit comporte au moins un orifice (1130).

31. Dispositif selon l'une des revendications 29 et 30, **caractérisé par le fait qu'**il est agencé de manière à rétablir automatiquement une communication fluidique entre ladite au moins une arrivée de produit et la réserve de produit, lors d'une émission de gaz.

32. Dispositif selon l'une quelconque des revendications précédentes, **caractérisé par le fait qu'**il comporte un bouton-poussoir apte à agir simultanément, directement ou indirectement, sur une valve de distribution de gaz et sur l'obturateur, de manière à ce qu'une communication fluidique entre ladite au moins une arrivée de produit et la réserve de produit soit établie quand l'utilisateur appuie sur le bouton-poussoir pour délivrer du gaz.

33. Dispositif selon l'une quelconque des revendications précédentes, **caractérisé par le fait qu'**il comporte un premier récipient (1104) fixé sur un deuxième récipient (101) contenant le gaz.

34. Dispositif selon l'une quelconque des revendications 29 à 34, **caractérisé par le fait que** le récipient contenant le produit et le récipient contenant le gaz sont liés fixement, sans déplacement de l'un par rapport à l'autre au cours de l'utilisation.

35. Dispositif selon l'une quelconque des revendications précédentes, **caractérisé par le fait que** le récipient (1104) contenant le produit présente une forme annulaire, afin de ménager un passage dans lequel peut s'étendre un organe de commande (120) d'une valve équipant le récipient contenant le gaz.

36. Dispositif selon l'une quelconque des revendications précédentes, **caractérisé par le fait que** l'obturateur (1150; 1201 ; 1202; 1210) est relié opérationnellement à au moins un élément actionné par le déplacement d'un bouton-poussoir du dispositif.

37. Dispositif selon la revendication 36, **caractérisé par le fait que** ledit élément comporte une tige creuse (1140), ayant au moins un canal intérieur permettant au produit de gagner ledit au moins un orifice d'arrivée de produit.

38. Dispositif selon l'une quelconque des revendications précédentes, **caractérise par le fait que** l'obturateur comporte au moins un joint (1150), apte à obturer au moins un orifice (1145) par lequel le produit peut s'écouler pour gagner ladite au moins une arrivée de produit et apte à libérer cet orifice au moins partiellement lors de la distribution du produit

39. Dispositif selon la revendication 38, **caractérisé par le fait que** l'obturateur est constitué d'un joint (1150) monté sur une tige creuse (1140), cette dernière étant fermée à son extrémité inférieure, le joint pouvant venir en appui par sa face supérieure contre un épaulement de la tige, cette dernière étant traversée par au moins un orifice d'admission de produit (1145) dont le diamètre est inférieur ou égal à l'épaisseur du joint (1150), ce dernier étant apte à venir en butée par sa face inférieure contre une paroi d'appui (1114), fixe, quand la tige creuse est enfoncée, de sorte que le joint est alors comprimé et dégage au moins partiellement l'orifice d'admission (1145),- afin de permettre un écoulement de produit en direction de ladite au moins une arrivée de produit.

40. Dispositif selon la revendication 39, **caractérisé par le fait que** le joint (1150) s'applique à sa périphérie contre la surface intérieure d'un conduit (1111) dans lequel la tige creuse (1140) peut se déplacer axialement, ce conduit pouvant communiquer librement avec l'extérieur.

41. Dispositif selon la revendication 39 ou 40, **caractérisé par le fait que** la tige creuse (1140) est actionnée par le déplacement d'un bouton-poussoir (1103) commandant l'émission de gaz.

42. Dispositif selon l'une quelconque des revendications 39 à 41, **caractérisé par le fait que** l'espace intérieur à la paroi d'appui (1114) contre laquelle le joint (1150) peut venir en butée par sa face inférieure communique avec le récipient contenant le produit, par l'intermédiaire d'une gorge annulaire (1107) réalisée dans une paroi de fond (1105c) dudit récipient.

43. Dispositif selon l'une quelconque des revendications précédentes, **caractérisé par le fait qu'**il comporte un bouton-poussoir (103) réalisé par assemblage d'une partie inférieure (1103b) et d'une partie supérieure (1103a).

44. Dispositif selon la revendication 43, **caractérisé par le fait que** la partie inférieure (1103b) est réalisée d'un seul tenant avec une tige d'actionnement (1120), s'étendant dans un passage du récipient contenant le produit.

45. Dispositif selon la revendication 44, **caractérise par le fait que** la tige (1121) de commande de la valve contenant le gaz est engagée dans la tige d'actionnement (1120).

46. Dispositif selon l'une quelconque des revendications 29 à 45, **caractérisé par le fait que** le gaz est émis par au moins un orifice de sortie de gaz (1127a ; 1127b).

47. Dispositif selon la revendication 46, **caractérisé par le fait que** le gaz vecteur est émis par au moins deux orifices (1I27a ; 1127b) de sortie de gaz, dont les positions respectives sont choisies de manière à ce que les jets de gaz émis par ces orifices se rencontrent

## Claims

1. A self-contained device of unitary structure (1; 101; 201; 1100) for spraying a cosmetic substance onto a keratinous medium, in particular the skin, the device comprising a supply of substance to be sprayed and a receptacle containing a gas, together with a valve making it possible, when actuated, to spray the substance, the substance containing solid particles and at least one jelling agent, in which device the supply of substance is contained in a receptacle different from the receptacle containing the gas or the substance is contained in a flexible bag placed inside the receptacle containing the gas.

2. A device according to claim 1, **characterized by** the fact that the jelling agent is selected so that it does not crystallize at ambient temperature in the medium containing it.

3. A device according to claim 2, **characterized by** the fact that the jelling agent is selected from agents not included in the following list:
- waxes, in particular hydrocarbon waxes or silicone waxes (alkyl or alkoxydimethicone);
- fatty acid and fatty alcohol esters, including cerides and sterides or glycerophosphocholine and fatty acid esters, such as phospholipids; and
- amino and fatty acids, in particular ceramides.

4. A device according to any preceding claim, **characterized by** the fact that the substance comprises more than 0,3% by weight of solid particles, preferably 5% to 70% by weight of solid particles, preferably 20% to 70%, more preferably still 30% to 60%, particularly when the substance is a foundation makeup, and by the fact that the jelling agent content lies in the range 0.01% to 10% by weight, and is preferably greater than or equal to 0.1%.

5. A device according to any preceding claim, **characterized by** the fact that the substance includes pigments.

6. A device according to the preceding claim, **characterized by** the fact that the pigments are selected from the following list: iron, titanium, and zinc oxide, organic varnishes, inorganic pearlescent agents, in particular mica, titanium, or titanium-mica, and mixtures thereof.

7. A device according to any preceding claim, **characterized by** the fact that the substance contains particles selected from the following list: organic powders, in particular particles of polyamide, of polyacrylate, of methyl methacrylate, of polyurethane, of polyethylene, of cross-linked polystyrene, and mixtures thereof, powders of mineral origin such as talc, clay, silica, boron nitride and mixtures thereof, powders of vegetable origin such as starch, powders of animal origin such as seashell powder, silicones in the form of beads of methylsesquioxane resin, fluorine-containing powders such as PTFE powders, and mixtures thereof.

8. A device according to any one of claims 1 to 7, **characterized by** the fact that the particles are of micrometer size (individual grain size).

9. A device according to any one of claims 1 to 7, **characterized by** the fact that the particles are of nanometer size.

10. A device according to any preceding claim, **characterized by** the fact that the jelling agent (s) used is/are hydrophilic.

11. A device according to any one of claims 1 to 9, **characterized by** the fact that the jelling agent (s) used is/are lipophilic.

12. A device according to claim 10, **characterized by** the fact that the substance contains at least one hydrophilic jelling agent selected from: hydrophilic jelling agents of polymeric nature such as jelling agents of polysaccharide type, in particular xanthan gum, gellan gum, guar gum, and derivatives thereof, cellulose and derivatives thereof, jelling agents of vinyl type, carboxyvinyl type, acrylic type, polyurethane, mineral type jelling agents of non-polymeric nature such as clays, in particular Veegum®, optionally modified Bentone®, and hydrophilic silicas, and mixtures thereof.

13. A device according to claim 11, **characterized by** the fact that the substance contains a lipophilic jelling agent selected from the following lipophilic jelling agents: polymer type jelling agents or "organo-jelling" type jelling agents, linear or cross-linked silicone polymers and derivatives thereof, polymers derived from silicones, acrylic silicones, polyamide type polycondensates, polysaccharides having hydrophobic chains, and polyurethanes, and mixtures thereof.

14. A device according to any preceding claim, **characterized by** the fact that the substance contains inorganic jelling agents optionally modified by organic groups in order to jell oils.

15. A device according to claim 10 or claim 11, **characterized by** the fact that the substance contains at least one dispersing agent and at least one jelling agent.

16. A device according to the preceding claim, **characterized by** the fact that the substance contains at least one hydrophilic dispersing agent selected from the following hydrophilic dispersing agents: dispersing agents having at least one anionic filler, in particular carboxylic dispersing agents, sulfonates, terpolymers of acrylic acid, polyaspartates, derivatives of maleic acid, and mixtures thereof.

17. A device according to claim 15, **characterized by** the fact that the substance contains at least one lipophilic dispersing agent selected from the following lipophilic dispersing agents: carboxylates, acrylics, hydrocarbons with a ring, in particular styrene, and hydrocarbons of the succinimide type.

18. A device according to any preceding claim, **characterized by** the fact that the supply of substance is contained in a receptacle that is sufficiently closed to enable the substance to be conserved at ambient temperature in the device when not in use for a length of time in excess of at least 1 month.

19. A device according to any preceding claim, **characterized by** the fact that the viscosity of the substance lies in the range 20 centipoises to 500 centipoises, with viscosity being measured using an RM 180 rheometer from Rheometric Scientific, of the rotary type, using a suitable moving unit of "1", "2", or "3" type depending on the fluidity of the formulation, after 10 minutes at a shear rate of 200/s, with measurements being performed at a temperature of 25°C.

20. A device according to any preceding claim, **characterized by** the fact that the substance contains hydrophilic or lipophilic active agents such as, for example, UV blockers (filters), and moisturizers, a care oil, an antioxidant, preserving agents, and anti-foaming agents.

21. A device according to any preceding claim, **characterized by** the fact that the supply of substance is contained in a removable cartridge.

22. A device according to any one of claims 1 to 20, **characterized by** the fact that the supply of substance is contained in a non-removable receptacle.

23. A device according to any preceding claim, **characterized by** the fact that it includes a tube dipping into the supply of substance.

24. A device according to any preceding claim, **characterized by** the fact that the supply of substance is contained in a receptacle which is different from the receptacle containing the gas.

25. A device according to any one of claims 1 to 11, **characterized by** the fact that the substance is contained in a flexible bag placed inside the receptacle containing the gas.

26. A device according to any preceding claim, **characterized by** the fact that the valve has at least three positions, namely: a rest position; a position enabling a mixture of substance and gas to be dispensed; and a position enabling the gas to be dispensed on its own.

27. A device according to any one of claims 1 to 24, **characterized by** the fact that it includes at least one substance feed duct (22) and at least one gas feed duct (21), preferably two gas feed ducts, the ducts being arranged in such a manner that the gas delivered by the feed duct generates a pressure reduction suitable for sucking substance from the substance feed duct.

28. A device according to any preceding claim, **characterized by** the fact that the gas is present in at least a liquid phase inside the receptacle (2) containing the gas.

29. A device according to claim 1, **characterized by** the fact that it comprises a supply of gas, at least one substance feed suitable for being put into fluid communication with a supply of said substance, the substance being sucked from the supply by suction established in the vicinity of said at least one substance feed by emission of said gas, and at least one shutter (1150; 1201) suitable for interrupting fluid communication between said at least one substance feed and the supply of substance.

30. A device according to claim 29, **characterized by** the fact that the substance feed comprises at least one orifice (1130).

31. A device according to claim 29 or claim 30, **characterized by** the fact that it is arranged in such a manner as to reestablish fluid communication automatically between said at least one substance feed and the supply of substance while gas is being emitted.

32. A device according to any preceding claim, **characterized by** the fact that it comprises a pushbutton suitable for acting simultaneously, directly or indirectly, on a gas dispenser valve and on the shutter, whereby fluid communication between said at least one substance feed and the supply of substance is established when the user presses on the pushbutton to cause the gas to be dispensed.

33. A device according to any preceding claim, **characterized by** the fact that it comprises a first receptacle (1104) fixed on a second receptacle (101) containing the gas.

34. A device according to any one of claims 29 to 34, **characterized by** the fact that the receptacle containing the substance and the receptacle containing the gas are fixed to each other in such a manner as to prevent them from moving relative to each other in use.

35. A device according to any preceding claim, **characterized by** the fact that the receptacle (1104) containing the substance is annular in shape so as to leave a passage through which a control member (120) can extend for controlling a valve fitted to the receptacle containing the gas.

36. A device according to any preceding claim, **characterized by** the fact that the shutter (1150; 1201; 1202; 1210) is operationally connected to at least one element actuated by moving the pushbutton of the device.

37. A device according to claim 36, **characterized by** the fact that said element comprises a hollow rod (1140) having at least one inside passage enabling the substance to reach said at least one substance feed orifice.

38. A device according to any preceding claim, **characterized by** the fact that the shutter comprises at least one gasket (1150) suitable for shutting at least one orifice (1145) through which the substance can flow to reach said at least one substance feed, and suitable for releasing said orifice at least in part while substance is being dispensed.

39. A device according to claim 38, **characterized by** the fact that the shutter is constituted by a gasket (1150) mounted on a hollow rod (1140), the rod being closed at its bottom end, the gasket being capable of bearing via its top face against a shoulder of the rod, the rod having at least one substance admission orifice (1145) passing therethrough, the orifice being of a diameter that is smaller than or equal to the thickness of the gasket (1150), said gasket being suitable for coming into abutment via its bottom face against a stationary bearing wall (1114) when the hollow rod is pushed down, thereby causing the gasket to be compressed so as to release the admission orifice (1145) at least in part, thus enabling substance to flow towards said at least one substance feed.

40. A device according to claim 39, **characterized by** the fact that the gasket (1150) bears via its periphery against the inside surface of a duct (1111) in which the hollow rod (1140) can move axially, the duct being capable of communicating freely with the outside.

41. A device according to claim 39 or claim 40, **characterized by** the fact that the hollow rod (1140) is actuated by moving a pushbutton (1103) that controls emission of the gas.

42. A device according to any one of claims 39 to 41, **characterized by** the fact that the space inside the bearing wall (1114) against which the gasket (1150) can come into abutment via its bottom face communicates with the receptacle containing the substance, via an annular channel (1107) formed in a bottom end wall (1105c) of said receptacle.

43. A device according to any preceding claim, **characterized by** the fact that it comprises a pushbutton (103) made by assembling together a bottom portion (1103b) and a top portion (1103a).

44. A device according to claim 43, **characterized by** the fact that the bottom portion (1103b) is made integrally with an actuator rod (1120) extending along a passage in the receptacle containing the substance.

45. A device according to claim 44, **characterized by** the fact that the control rod (1121) of the valve for delivering the gas is engaged in the actuator rod (1120).

46. A device according to any one of claims 29 to 45, **characterized by** the fact that the gas is emitted via at least one gas outlet orifice (1127a; 1127b).

47. A device according to claim 46, **characterized by** the fact that the gas is emitted through at least two gas outlet orifices (1127a; 1127b) whose respective positions are selected in such a manner that the gas jets emitted by the orifices meet.

## Patentansprüche

1. Autonome Vorrichtung (1; 101; 201; 1100) von einheitlichem-Aufbau zum Aufstäuben eines kosmetischen Produkts auf einen Keratinträger, insbesondere die Haut, umfassend eine Reserve von zu zerstäubendem Produkt und einen ein Gas enthaltenden Behälter sowie ein Ventil, das, wenn es betätigt wird, die Zerstäubung des Produkts gestattet, wobei dieses feste Teilchen und mindestens einen Gelbildner enthält, wobei in dieser Vorrichtung die Produktreserve in einem anderen Behälter als demjenigen, der das Gas enthält, enthalten ist oder Produkt in einem biegsamen Beutel enthalten ist, der im Inneren des das Gas enthaltenden Behälters angeordnet ist.

2. Vorrichtung nach Anspruch 1, **dadurch gekennzeichnet, dass** der Gelbildner so gewählt ist, dass er in dem es enthaltenden Medium bei Umgebungstemperatur nicht kristallisiert.

3. Vorrichtung nach Anspruch 2, **dadurch gekennzeichnet, dass** der Gelbildner außerhalb der folgenden Liste ausgewählt ist:
- Wachse, insbesondere Kohlenwasserstoffwachse oder Siliconwachse (Alkyl oder Alkoxydimeticon),
- Ester aus Fettalkohol und Fettsäure, darunter Ceride und Steride oder Ester aus Fettsäure und Glycerophoscholin, wie Phospholipide,
- Fett- und Aminosäuren, insbesondere Ceramide.

4. Vorrichtung nach einem der vorhergehenden Ansprüche, **dadurch gekennzeichnet, dass** das Produkt mehr als 0,3 Gew.-% feste Teilchen, vorzugsweise zwischen 5 und 70 Gew.-% feste Teilchen, vorzugsweise zwischen 20 und 70 %, noch bevorzugter zwischen 30 und 60 % umfasst, insbesondere wenn das Produkt eine Grundierung ist, und **dadurch**, dass der Gelbildner in einem Anteil zwischen 0,01 und 10 Gew.-%, vorzugsweise in einem Anteil größer als oder gleich 0,1 %, vorliegt.

5. Vorrichtung nach einem der vorhergehenden Ansprüche, **dadurch gekennzeichnet, dass** das Produkt Pigmente umfasst.

6. Vorrichtung nach dem vorhergehenden Anspruch, **dadurch gekennzeichnet, dass** die Pigmente aus der folgenden Liste ausgewählt sind: Eisen-, Titan- oder Zinkoxid, organische Lacke, mineralische Perlmuttstoffe insbesondere aus Glimmer, Titan oder Glimmertitan und ihren Mischungen.

7. Vorrichtung nach einem der vorhergehenden Ansprüche, **dadurch gekennzeichnet, dass** das Produkt Teilchen umfasst, die aus der folgenden Liste ausgewählt sind: organische Pulver, insbesondere Teilchen aus Polyamid, Polyacrylat, Methylmethacrylat, Polyurethan, Polyethylen, vernetztes Polystyrol oder ihre Mischungen, Pulver mineralischen Ursprungs, wie Talk, Ton, Siliciumoxid, Bornitrid oder ihre Mischungen, Pulver pflanzlichen Ursprungs, wie Stärke, Pulver tierischen Ursprungs, wie Pulver von Muscheln, Silicone in Form von Kugeln aus Methylsesquioxanharz, fluorierte Pulver wie PTFE-Pulver, und ihre Mischungen.

8. Vorrichtung nach einem der Ansprüche 1 bis 7, **dadurch gekennzeichnet, dass** die Teilchen von mikrometrischer Größe sind (Größe des Einzelkorns).

9. Vorrichtung nach einem der Ansprüche 1 bis 7, **dadurch gekennzeichnet, dass** die Teilchen von nanometrischer Größe sind.

10. Vorrichtung nach einem der vorhergehenden Ansprüche, **dadurch gekennzeichnet, dass** der oder die verwendeten Gelbildner hydrophil sind.

11. Vorrichtung nach einem der Ansprüche 1 bis 9, **dadurch gekennzeichnet, dass** der oder die verwendeten Gelbildner lipophil sind.

12. Vorrichtung nach Anspruch 10, **dadurch gekennzeichnet, dass** das Produkt mindestens einen hydrophilen Gelbildner umfasst, der ausgewählt ist aus: hydrophile Gelbildner polymerischer Natur, wie Gelbildner vom Typ Polysaccharid, insbesondere Xanthan-, Gellan-, Guargummi oder seine Derivate, Cellulose und ihre Derivate, Gelbildner vom vinylischen, carboxyvinylischen, acrylischen Typ, Polyurethan, Gelbildner nichtpolymerischer Natur vom mineralischen Typ wie Tone, insbesondere Veegum®, modifiziertes oder nichtmodifiziertes Bentone® oder hydrophile Siliciumoxide oder ihre Mischungen.

13. Vorrichtung nach Anspruch 11, **dadurch gekennzeichnet, dass** das Produkt einen lipophilen Gelbildner umfasst, der aus den folgenden lipophilen Gelbildnern ausgewählt ist: Gelbildner vom polymerischen Typ oder vom Typ "Organo-Gelbildner", lineare oder vernetzte Siliconpolymere und ihre Derivate, von den Siliconen abgeleitete Polymere, Acrylsilicone, Polykondensate vom Typ Polyamide, Polysaccharide mit hydrophoben Ketten oder Polyurethane oder ihre Mischungen.

14. Vorrichtung nach einem der vorhergehenden Ansprüche, **dadurch gekennzeichnet, dass** das Produkt mineralische Gelbildner umfasst, die gegebenenfalls durch organische Gruppen modifiziert sein können, um Öle zu gelieren.

15. Vorrichtung nach den Ansprüchen 10 oder 11, **dadurch gekennzeichnet, dass** das Produkt sowohl mindestens ein Dispersionsmittel als auch mindestens einen Gelbildner umfasst.

16. Vorrichtung nach dem vorhergehenden Anspruch, **dadurch gekennzeichnet, dass** das Produkt mindestens ein hydrophiles Dispersionsmittel umfasst, das aus den folgenden hydrophilen Dispersionsmitteln ausgewählt ist: Dispersionsmittel mit mindestens einem anionischen Füllstoff, insbesondere carboxylische Dispersionsmittel, Sulfonate, Terpolymere der Acrylsäure, Polyaspartate, Derivate der Maleinsäure oder ihre Mischungen.

17. Vorrichtung nach Anspruch 15, **dadurch gekennzeichnet, dass** das Produkt mindestens ein lipophiles Dispersionsmittel umfasst, das aus den folgenden lipophilen Dispersionsmitteln ausgewählt ist: Carboxylate, Acryle, Kohlenwasserstoffe, mit Ring, insbesondere Styrol, oder vom Typ Succinimid.

18. Vorrichtung nach einem der vorhergehenden Ansprüche, **dadurch gekennzeichnet, dass** die Produktreserve in einem Behälter enthalten ist, der ausreichend geschlossen ist, um eine Haltbarkeit des Produkts in der Vorrichtung bei Nichtverwendung von mehr als mindestens einem Monat bei Raumtemperatur zu gestatten.

19. Vorrichtung nach einem der vorhergehenden Ansprüche, **dadurch gekennzeichnet, dass** die Viskosität des Produkts zwischen 20 Centipoise und 500 Centipoise beträgt, gemessen mit Hilfe eines rotierenden Rheomats RM 180 der Firma RHEOMETRIC SCIENTIFIC, der ein adaptiertes bewegliches Element vom sogenannten Typ "1", "2" oder "3" je nach der Fließfähigkeit der Formulierung verwendet, nach zehn Minuten eines Schergrads von 200/s, wobei die Temperatur der Messungen 25°C beträgt.

20. Vorrichtung nach einem der vorhergehenden Ansprüche, **dadurch gekennzeichnet, dass** das Produkt hydrophile oder lipophile Wirkstoffe wie beispielsweise UV-Blocker (Filter) oder Hydratisierungsmittel, ein Pflegeöl, ein Antioxidans, Konservierungsmittel oder Antischaummittel umfasst.

21. Vorrichtung nach einem der vorhergehenden Ansprüche, **dadurch gekennzeichnet, dass** die Produktreserve in einer abnehmbaren Kartusche enthalten ist.

22. Vorrichtung nach einem der Ansprüche 1 bis 20, **dadurch gekennzeichnet, dass** die Produktreserve in einem nicht abnehmbaren Behälter enthalten ist.

23. Vorrichtung nach einem der vorhergehenden Ansprüche, **dadurch gekennzeichnet, dass** sie ein in die Produktreserve eintauchendes Rohr umfasst.

24. Vorrichtung nach einem der vorhergehenden Ansprüche, **dadurch gekennzeichnet, dass** die Produktreserve in einem Behälter enthalten ist, der von demjenigen, der das Gas enthält, verschieden ist.

25. Vorrichtung nach einem der Ansprüche 1 bis 11, **dadurch gekennzeichnet, dass** das Produkt in einem biegsamen Beutel enthalten ist, der im Inneren des das Gas enthaltenden Behälters angeordnet ist.

26. Vorrichtung nach einem der vorhergehenden Ansprüche, **dadurch gekennzeichnet, dass** das Ventil mindestens drei Positionen hat, und zwar eine Ruhestellung, eine Stellung, die die Abgabe einer Mischung von Produkt und Gas gestattet, und eine Stellung, die die Abgabe nur von Gas gestattet.

27. Vorrichtung nach einem der Ansprüche 1 bis 24, **dadurch gekennzeichnet, dass** sie mindestens eine Produktzufuhrleitung (22) und mindestens eine Gaszufuhrleitung (21), vorzugsweise zwei, umfasst, wobei die Leitungen so ausgebildet sind, dass der Austritt von Gas über die Zufuhrleitung einen Unterdruck bewirkt, der geeignet ist, durch Ansaugung von der Produktzufuhrleitung kommendes Produkt mitzunehmen.

28. Vorrichtung nach einem der vorhergehenden Ansprüche, **dadurch gekennzeichnet, dass** das Gas im Inneren des das Gas enthaltenden Behälters (2) mindestens in einer flüssigen Phase vorliegt.

29. Vorrichtung nach Anspruch 1, **dadurch gekennzeichnet, dass** sie eine Gasreserve, mindestens eine Produktzufuhr, die geeignet ist, mit einer Reserve dieses Produkts in Fluidverbindung gebracht zu werden, wobei letzteres in der Reserve durch einen Unterdruck angesaugt wird, der in der Nähe dieser mindestens einen Produktzufuhr mit Hilfe einer Emission dieses Gases erzeugt wird, und mindestens ein Verschlussorgan (1150; 1201) umfasst, das geeignet ist, die Fluidverbindung zwischen der mindestens einen Produktzufuhr und der Produktreserve zu unterbrechen.

30. Vorrichtung nach Anspruch 29, **dadurch gekennzeichnet, dass** die Produktzufuhr mindestens eine Öffnung (1130) umfasst.

31. Vorrichtung nach einem der Ansprüche 29 und 30, **dadurch gekennzeichnet, dass** sie so ausgebildet ist, dass bei einer Gasemission zwischen der mindestens einen Produktzufuhr und der Produktreserve automatisch eine Fluidverbindung wieder hergestellt wird.

32. Vorrichtung nach einem der vorhergehenden Ansprüche, **dadurch gekennzeichnet, dass** sie einen Druckknopf umfasst, der geeignet ist, gleichzeitig, direkt oder indirekt, auf ein Gasabgabeventil und auf das Verschlussorgan so einzuwirken, dass zwischen der mindestens einen Produktzufuhr und der Produktreserve eine Fluidverbindung hergestellt wird, wenn der Benutzer auf den Druckknopf drückt, um Gas abzugeben.

33. Vorrichtung nach einem der vorhergehenden Ansprüche, **dadurch gekennzeichnet, dass** sie einen ersten Behälter (1104) umfasst, der auf einem zweiten, das Gas enthaltenden Behälter (101) befestigt ist.

34. Vorrichtung nach einem der Ansprüche 29 bis 34, **dadurch gekennzeichnet, dass** der das Produkt enthaltende Behälter und der das Gas enthaltende Behälter fest ohne Bewegung des einen bezüglich des anderen bei der Benutzung verbunden sind.

35. Vorrichtung nach einem der vorhergehenden Ansprüche, **dadurch gekennzeichnet, dass** der das Produkt enthaltende Behälter (1104) eine Ringform besitzt, um einen Durchgang zu bilden, in dem sich ein Organ (120) zur Betätigung eines Ventils erstrecken kann, mit dem der das Gas enthaltende Behälter ausgerüstet ist.

36. Vorrichtung nach einem der vorhergehenden Ansprüche, **dadurch gekennzeichnet, dass** das Verschlussorgan (1150; 1201; 1202; 1210) operationell mit mindestens einem durch die Bewegung eines Druckknopfs der Vorrichtung betätigten Element verbunden ist.

37. Vorrichtung nach Anspruch 36, **dadurch gekennzeichnet, dass** das Element eine hohle Stange (1140) umfasst, die mindestens einen inneren Kanal besitzt, der dem Produkt gestattet, zu der mindestens einen Produktzufuhröffnung zu gelangen.

38. Vorrichtung nach einem der vorhergehenden Ansprüche, **dadurch gekennzeichnet, dass** das Verschlussorgan mindestens eine Dichtung (1150) umfasst, die geeignet ist, mindestens eine Öffnung (1145) zu verschließen, über die das Produkt fließen kann, um die mindestens eine Produktzufuhr zu erreichen, und geeignet ist, diese Öffnung bei der Abgabe des Produkts mindestens teilweise freizugeben.

39. Vorrichtung nach Anspruch 38, **dadurch gekennzeichnet, dass** das Verschlussorgan aus einer Dichtung (1150) besteht, die auf einer hohlen Stange (1140) montiert ist, wobei diese an ihrem unteren Ende geschlossen ist, wobei die Dichtung mit ihrer Oberseite an einer Schulter der Stange zum Anliegen kommen kann, wobei letztere von mindestens einer Produkteinlassöffnung (1145) durchsetzt ist, deren Durchmesser kleiner als oder gleich der Dicke der Dichtung (1150) ist, wobei letztere geeignet ist, mit ihrer Unterseite an einer feststehenden Auflagewand (1114) in Anschlag zu kommen, wenn die hohle Stange eingedrückt ist, so dass die Dichtung nun komprimiert ist und die Einlassöffnung (1145) mindestens teilweise freigibt, um eine Produktströmung in Richtung dieser mindestens einen Produktzufuhr zu gestatten.

40. Vorrichtung nach Anspruch 39, **dadurch gekennzeichnet, dass** die Dichtung (1150) sich an ihrem Umfang an die Innenseite einer Leitung (1111) anlegt, in der die hohle Stange (1140) sich axial bewegen kann, wobei diese Leitung mit dem Äußeren frei verbunden sein kann.

41. Vorrichtung nach Anspruch 39 oder 40, **dadurch gekennzeichnet, dass** die hohle Stange (1140) durch die Bewegung eines die Gasemission steuernden Druckknopfs (1103) betätigt wird.

42. Vorrichtung nach einem der Ansprüche 39 bis 41, **dadurch gekennzeichnet, dass** der Raum innerhalb der Auflagewand (1114), an der die Dichtung (1150) mit ihrer Unterseite in Anschlag kommen kann, mit dem das Produkt enthaltenden Behälter über eine ringförmige Nut (1107) in Verbindung ist, die in einer Bodenwand (1105c) des Behälters gebildet ist.

43. Vorrichtung nach einem der vorhergehenden Ansprüche, **dadurch gekennzeichnet, dass** sie einen Druckknopf (103) umfasst, der durch Zusammenfügung eines unteren Teils (1103b) und eines oberen Teils (1103a) gebildet ist.

44. Vorrichtung nach Anspruch 43, **dadurch gekennzeichnet, dass** der untere Teil (1103b) einstückig mit einer Betätigungsstange (1120) gebildet ist, die sich in einem Durchgang des das Produkt enthaltenden Behälters erstreckt.

45. Vorrichtung nach Anspruch 44, **dadurch gekennzeichnet, dass** die Stange (1121) zur Steuerung des das Gas enthaltenden Ventils in die Betätigungsstange (120) eingeführt ist.

46. Vorrichtung nach einem der Ansprüche 29 bis 45, **dadurch gekennzeichnet, dass** das Gas über mindestens eine Gasaustrittsöffnung (1127a; 1127b) abgegeben wird.

47. Vorrichtung nach Anspruch 46, **dadurch gekennzeichnet, dass** das Trägergas über mindestens zwei Gasaustrittsöffnungen (1127a; 1127b) abgegeben wird, deren jeweilige Stellungen so gewählt sind, dass die von diesen Öffnungen abgegebenen Gasstrahlen sich treffen.
